(19) Europäisches Patentamt
European Patent Office
Office européen des brevets

(11) Veröffentlichungsnummer: **0 476 607 A2**

# (12) EUROPÄISCHE PATENTANMELDUNG

(21) Anmeldenummer: **91115827.7**

(51) Int. Cl.[5]: **A01N 43/40**, C07D 213/84

(22) Anmeldetag: **18.09.91**

(30) Priorität: **20.09.90 DE 4029772**

(43) Veröffentlichungstag der Anmeldung:
**25.03.92 Patentblatt 92/13**

(84) Benannte Vertragsstaaten:
**CH DE DK ES FR GB IT LI NL**

(71) Anmelder: **BASF Aktiengesellschaft**
**Carl-Bosch-Strasse 38**
**W-6700 Ludwigshafen(DE)**

(72) Erfinder: **Schubert, Juergen, Dr.**
**Am oberen Luisenpark 2**
**W-6800 Mannheim 1(DE)**
Erfinder: **Kardorff, Uwe, Dr.**
**D 3,4**
**W-6800 Mannheim 1(DE)**
Erfinder: **Kuenast, Christoph, Dr.**
**Salierstrasse 2**
**W-6701 Otterstadt(DE)**

## (54) 2-Anilino-cyanopyridine und ihre Verwendung zur Bekämpfung von Schädlingen.

(57) Verfahren zu Bekämpfung von Schädlingen mittels eines 2-Anilino-cyanopyridins der allgemeinen Formel I,

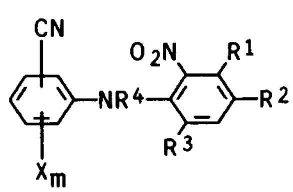

I

in der die Substituenten und der Index die folgende Bedeutung haben:

| | |
|---|---|
| X | Nitro; Cyano; Halogen; Alkyl; Halogenalkyl; Alkoxy; Cycloalkyl; Alkylsulfonyl; ggf. subst. Phenyl, Phenoxy oder Phenylthio, |
| m | 1, 2, oder 3, |
| $R^1$ | Wasserstoff; Halogen; Alkyl; Halogenalkyl; Alkoxy; Halogenalkoxy; ggf. subst. Phenoxy oder Phenylthio, |
| $R^2$ und $R^3$ | unabhängig voneinander Wasserstoff; Nitro; Cyano; Carboxyl; Halogen; Alkyl; Halogenalkyl; Alkoxy; Halogenalkoxy; Alkylthio; Halogenalkylthio; Alkoxycarbonyl; Alkylaminocarbonyl; Dialkylaminocarbonyl; Alkylsulfonyl; Alkylaminosulfonyl; Dialkylaminosulfonyl; |
| $R^4$ | Wasserstoff; Formyl; Alkyl; Halogenalkyl; Alkenyl; Alkinyl; ggf. subst. Benzyl; $COR^5$ oder $SO_2R^6$; |
| $R^5$ | Alkyl; Halogenalkyl; Alkoxy; Halogenalkoxy; Alkylthio; Halogenalkylthio; Alkylamino; Dialkylamino; ggf. subst. Benzyl, Benzyloxy, Benzylthio oder Benzylamino; |
| $R^6$ | Alkyl; Halogenalkyl; ggf. subst. Phenyl oder Benzyl; |

sowie deren landwirtschaftlich brauchbaren Salze und die Verwendung dieser Verbindungen.

EP 0 476 607 A2

Die vorliegende Erfindung betrifft Verfahren zu Bekämpfung von Schädlingen, welche dadurch gekennzeichnet sind, daß man die Schädlinge und/oder ihren Lebensraum mit einer wirksamen Menge eines 2-Anilino-cyanopyridins der allgemeinen Formel I,

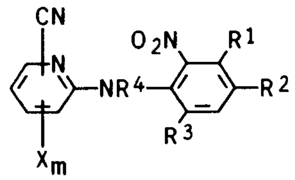

I

behandelt, in der die Substituenten und der Index die folgende Bedeutung haben:

X Nitro; Cyano; Halogen; $C_1$-$C_4$-Alkyl; $C_1$-$C_4$-Halogenalkyl; $C_1$-$C_4$-Alkoxy; $C_3$-$C_6$-Cycloalkyl; $C_1$-$C_4$-Alkylsulfonyl; Phenyl, Phenoxy oder Phenylthio, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

m 1, 2, oder 3, wobei die Reste verschieden sein können, wenn m für 2 oder 3 steht;

$R^1$ Wasserstoff; Halogen; $C_1$-$C_4$-Alkyl; $C_1$-$C_4$-Halogenalkyl; $C_1$-$C_4$-Alkoxy; $C_1$-$C_4$-Halogenalkoxy; Phenoxy oder Phenylthio, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^2$ und $R^3$ unabhängig voneinander Wasserstoff; Nitro; Cyano; Carboxyl; Halogen; $C_1$-$C_6$-Alkyl; $C_1$-$C_6$-Halogenalkyl; $C_1$-$C_4$-Alkoxy; $C_1$-$C_4$-Halogenalkoxy; $C_1$-$C_4$-Alkylthio; $C_1$-$C_4$-Halogenalkylthio; $C_1$-$C_4$-Alkoxycarbonyl; $C_1$-$C_4$-Alkylaminocarbonyl; Di-$C_1$-$C_4$-alkylaminocarbonyl; $C_1$-$C_4$-Alkylsulfonyl; $C_1$-$C_4$-Alkylaminosulfonyl; Di-$C_1$-$C_4$-alkylaminosulfonyl;

$R^4$ Wasserstoff; Formyl; $C_1$-$C_6$-Alkyl; $C_1$-$C_6$-Halogenalkyl; $C_3$-$C_6$-Alkenyl; $C_3$-$C_6$-Alkinyl; Benzyl, welches seinerseits ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio; $COR^5$ oder $SO_2R^6$, wobei

$R^5$ für $C_1$-$C_6$-Alkyl; $C_1$-$C_6$-Halogenalkyl; $C_1$-$C_4$-Alkoxy; $C_1$-$C_4$-Halogenalkoxy; $C_1$-$C_4$-Alkylthio; $C_1$-$C_4$-Halogenalkylthio; $C_1$-$C_4$-Alkylamino; Di-$C_1$-$C_4$-alkylamino; Benzyl, Benzyloxy, Benzylthio oder Benzylamino steht, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

und

$R^6$ für $C_1$-$C_6$-Alkyl; $C_1$-$C_6$-Halogenalkyl; Phenyl oder Benzyl steht, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio,

sowie deren landwirtschaftlich brauchbaren Salze sowie die Verwendung dieser Verbindungen.

Die eingangs definierten 2-Anilino-cyanopyridine der Formel I sind aus der DE-A 39 25 238 als fungizid wirksam bekannt. Außerdem werden in der Literatur 2-Anilino-pyridine mit fungiziden und insektiziden Eigenschaften beschrieben (EP-A 31 257).

Der Erfindung lagen neue Verfahren zur Bekämpfung von Insekten als Aufgabe zugrunde.

Demgemäß wurden das eingangs definierte Verfahren und die Verwendung der 2-Anilino-cyanopyridine der Formel I gefunden.

Man erhält die Verbindungen I beispielsweise nach den in der eingangs zitierten Literatur beschriebenen Verfahren. Im Hinblick auf die bestimmungsgemäße Verwendung der Verbindungen I kommen als Substituenten folgende Reste in Betracht:

X Nitro; Cyano;
Halogen wie Fluor, Chlor, Brom und Jod, vorzugsweise Fluor, Chlor und Brom, insbesondere Chlor und Brom;
$C_1$-$C_4$-Alkyl wie Methyl, Ethyl, Propyl, 1-Methylethyl, Butyl, 1-Methyl-propyl, 2-Methylpropyl und 1,1-Dimethylethyl, vorzugsweise $C_1$-$C_3$-Alkyl, insbesondere Methyl, Ethyl und 1-Methylethyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Chlormethyl, Dichlormethyl, Trichlormethyl, Trifluormethyl und Pentafluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise $C_1$-$C_3$-Alkoxy, insbesondere Methoxy und Ethoxy;

$C_3$-$C_6$-Cycloalkyl wie Cyclopropyl, Cyclobutyl, Cyclopentyl und Cyclohexyl, insbesondere Cyclopropyl;

$C_1$-$C_4$-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl und 1,1-Dimethylethylsulfonyl, vorzugsweise $C_1$-$C_3$-Alkylsulfonyl, insbesondere Methylsulfonyl;

Phenyl, Phenoxy oder Phenylthio, wobei die aromatischen Gruppen ihrerseits ein bis fünf Halogenatome wie Fluor, Chlor, Brom und Jod, vorzugsweise Fluor, Chlor und Brom, insbesondere Chlor;

und/oder ein bis drei der folgenden Reste tragen können:

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise $C_1$-$C_3$-Alkyl, insbesondere Methyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy und Ethoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Trichlormethoxy und Trifluormethoxy;

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio und Ethylthio;

und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;

m 1, 2, oder 3, wobei die Reste verschieden sein können, wenn m für 2 oder 3 steht;

$R^1$ Wasserstoff;

Halogen wie Fluor, Chlor, Brom und Jod, vorzugsweise Fluor, Chlor und Brom, insbesondere Fluor und Chlor;

$C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, Vorzugsweise $C_1$-$C_3$-Alkoxy, insbesondere Methoxy und Ethoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy;

Phenoxy oder Phenylthio, wobei die aromatischen Gruppen ihrerseits ein bis fünf Halogenatome wie Fluor, Chlor, Brom und Jod, vorzugsweise Fluor, Chlor und Brom, insbesondere Fluor und Chlor;

und/oder ein bis drei der folgenden Reste tragen können:

$C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy und Ethoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethyloxy;

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;

und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;

$R^2$ und $R^3$ unabhängig voneinander Wasserstoff; Nitro; Cyano; Carboxyl;

Halogen wie Fluor, Chlor, Brom und Jod, vorzugsweise Fluor, Chlor und Brom, insbesondere Fluor und Chlor;

$C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_4$-Alkyl, insbesondere Methyl, Ethyl, 1-Methylethyl, 1-Methylpropyl und 1,1-Dimethylethyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl und Trichlormethyl, insbesondere Trifluormethyl;

$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise $C_1$-$C_3$-Alkoxy, insbesondere Methoxy und Ethoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy;
$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise $C_1$-$C_3$-Alkylthio, insbesondere Methylthio und Ethylthio;
und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;
$C_1$-$C_4$-Alkoxycarbonyl wie Methoxycarbonyl, Ethoxycarbonyl, Propyloxycarbonyl, 1-Methylethoxycarbonyl, Butyloxycarbonyl, 1-Methyl-propyloxycarbonyl, 2-Methylpropyloxycarbonyl und 1,1-Dimethylethoxycarbonyl, vorzugsweise $C_1$-$C_3$-Alkoxycarbonyl, insbesondere Methoxycarbonyl und Ethoxycarbonyl;
$C_1$-$C_4$-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl und 1-Ethyl-2-methylpropylaminocarbonyl, vorzugsweise $C_1$-$C_3$-Alkylaminocarbonyl, insbesondere $C_1$-$C_2$-Alkylaminocarbonyl;
Di-$C_1$-$C_4$-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl,N-Methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methyl-ethyl)aminocarbonyl, N-Butyl-N-ethylaminocarbonyl, N-Ethyl-N-(1-methylpropyl)-aminocarbonyl, N-Ethyl-N-(2-methylpropyl)aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methyl-ethyl)aminocarbonyl, N-(1-Methylethyl)-N-(1-methylpropyl)aminocarbonyl, N-(1-Methylethyl)-N-(2-methylpropyl)-aminocarbonyl, N-(1,1-Dimethylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl und N-(1,1-Dimethylethyl)-N-(2-methylpropyl)aminocarbonyl, vorzugsweise Di-$C_1$-$C_2$-Alkylaminocarbonyl, insbesondere Dimethylaminocarbonyl;
$C_1$-$C_4$-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl, vorzugsweise $C_1$-$C_3$-Alkylsulfonyl, insbesondere Methylsulfonyl;
$C_1$-$C_4$-Alkylaminosulfonyl wie Methylaminosulfonyl, Ethylaminosulfonyl, Propylaminosulfonyl, 1-Methylethylaminosulfonyl, Butylaminosulfonyl, 1-Methylpropylaminosulfonyl, 2-Methylpropylaminosulfonyl, 1,1-Dimethylethylaminosulfonyl, Pentylaminosulfonyl, 1-Methylbutylaminosulfonyl, 2-Methylbutylaminosulfonyl, 3-Methylbutylaminosulfonyl, 2,2-Dimethylpropylaminosulfonyl, 1-Ethylpropylaminosulfonyl, Hexylaminosulfonyl, 1,1-Dimethylpropylaminosulfonyl, 1,2-Dimethylpropylaminosulfonyl, 1-Methylpentylaminosulfonyl, 2-Methylpentylaminosulfonyl, 3-Methylpentylaminosulfonyl, 4-Methylpentylaminosulfonyl, 1,1-Dimethylbutylaminosulfonyl, 1,2-Dimethylbutylaminosulfonyl, 1,3-Dimethylbutylaminosulfonyl,

2,2-Dimethylbutylaminosulfonyl, 2,3-Dimethylbutylaminosulfonyl, 3,3-Dimethylbutylaminosulfonyl, 1-Ethylbutylaminosulfonyl, 2-Ethylbutylaminosulfonyl, 1,1,2-Trimethylpropylaminosulfonyl, 1,2,2-Trimethylpropylaminosulfonyl, 1-Ethyl-1-methylpropylaminosulfonyl und 1-Ethyl-2-methylpropylaminosulfonyl, vorzugsweise $C_1$-$C_3$-Alkylaminosulfonyl, insbesondere Methylaminosulfonyl;

Di-$C_1$-$C_4$-alkylaminosulfonyl wie N,N-Dimethylaminosulfonyl, N,N-Diethylaminosulfonyl, N,N-Dipropylaminosulfonyl, N,N-Di-(1-methylethyl)aminosulfonyl, N,N-Dibutylaminosulfonyl, N,N-Di-(1-methylpropyl)aminosulfonyl, N,N-Di-(2-methylpropyl)aminosulfonyl, N,N-Di-(1,1-dimethylethyl)aminosulfonyl, N-Ethyl-N-methylaminosulfonyl,N-Methyl-N-propylaminosulfonyl, N-Methyl-N-(1-methylethyl)aminosulfonyl, N-Butyl-N-methylaminosulfonyl, N-Methyl-N-(1-methylpropyl)aminosulfonyl, N-Methyl-N-(2-methylpropyl)aminosulfonyl, N-(1,1-Dimethylethyl)-N-methylaminosulfonyl, N-Ethyl-N-propylaminosulfonyl, N-Ethyl-N-(1-methyl-ethyl)aminosulfonyl, N-Butyl-N-ethylaminosulfonyl, N-Ethyl-N-(1-methylpropyl)-aminosulfonyl, N-Ethyl-N-(2-methylpropyl)aminosulfonyl, N-Ethyl-N-(1,1-dimethylethyl)-aminosulfonyl, N-(1-Methylethyl)-N-propylaminosulfonyl, N-Butyl-N-propylaminosulfonyl, N-(1-Methylpropyl)-N-propylaminosulfonyl, N-(2-Methylpropyl)-N-propylaminosulfonyl, N-(1,1-Dimethylethyl)-N-propylaminosulfonyl, N-Butyl-N-(1-methyl-ethyl)aminosulfonyl, N-(1-Methylethyl)-N-(1-methylpropyl)aminosulfonyl, N-(1-Methylethyl)-N-(2-methylpropyl)-aminosulfonyl, N-(1,1-Di-methylethyl)-N-(1-methylethyl)aminosulfonyl, N-Butyl-N-(1-methylpropyl)aminosulfonyl, N-Butyl-N-(2-methylpropyl)aminosulfonyl, N-Butyl-N-(1,1-dimethylethyl)aminosulfonyl, N-(1-Methylpropyl)-N-(2-methyl-propyl)aminosulfonyl, N-(1,1-Dimethylethyl)-N-(1-methylpropyl)aminosulfonyl und N-(1,1-Dimethylethyl)-N-(2-methylpropyl)aminosulfonyl, insbesondere Dimethylaminosulfonyl;

$R^4$ Wasserstoff; Formyl;

$C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_4$-Alkyl, insbesondere $C_1$-$C_2$-Alkyl;

$C_1$-$C_6$-Halogenalkyl, besonders $C_1$-$C_4$-Halogenalkyl, insbesondere $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl, 2,2,2-Trichlorethyl und 2,2,2-Trifluorethyl;

$C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl; vorzugsweise $C_3$-$C_4$-Alkenyl, insbesondere 2-Propenyl, 2-Butenyl und 3-Butenyl;

$C_3$-$C_6$-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl,1-Ethyl-3-butinyl, 2-Ethyl-3-butinyl und 1-Ethyl-1-methyl-2-propinyl; vorzugsweise $C_3$-$C_4$-Alkinyl, insbesondere 2-Propinyl;

Benzyl, welches seinerseits ein bis fünf Halogenatome wie Fluor, Chlor, Brom und Jod, vorzugsweise Fluor und Chlor;
und/oder ein bis drei der folgenden Reste tragen kann:
$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise $C_1$-$C_2$-Alkyl, insbesondere Methyl;
$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl;
$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy und Ethoxy;
$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy;
$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio und Ethylthio;
und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;
$R^4$ steht außerdem für $COR^5$ oder $SO_2R^6$, wobei $R^5$ für
$C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_4$-Alkyl, insbesondere Methyl, Ethyl, Propyl und 1,1-Dimethylethyl;
$C_1$-$C_6$-Halogenalkyl, besonders $C_1$-$C_4$-Halogenalkyl wie insbesondere Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy und Ethoxy;
$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy;
$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio und Ethylthio;
$C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;

$C_1$-$C_4$-Alkylamino wie Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino, vorzugsweise $C_1$-$C_3$-Alkylamino, insbesondere $C_1$-$C_2$-Alkylamino;

Di-$C_1$-$C_4$-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methyl-ethyl)amino, N-Butyl-N-ethylamino, N-Ethyl-N-(1-methyl-propyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methyl-ethyl)amino, N-(1-Methylethyl)-N-(1-methylpropyl)amino, N-(1-Methylethyl)-N-(2-methylpropyl)amino, N-(1,1-Di-methylethyl)-N-(1-methylethyl)amino, N-Butyl-N-(1-methyl-propyl)amino, N-Butyl-N-(2-methyl-propyl)amino, N-Butyl-N-(1,1-dimethylethyl)amino, N-(1-Methyl-propyl)-N-(2-methyl-propyl)amino, N-(1,1-Dimethylethyl)-N-(1-methyl-propyl)amino und N-(1,1-Dimethylethyl)-N-(2-methylpropyl)amino, vorzugsweise Di-$C_1$-$C_2$-Alkylamino, insbesondere Dimethylamino;

Benzyl, Benzyloxy, Benzylthio oder Benzylamino steht, wobei die aromatischen Gruppen ihrerseits ein bis fünf Halogenatome wie Fluor, Chlor, Brom und Jod, vorzugsweise Fluor, Chlor und Brom, insbesondere Fluor und Chlor;

und/oder ein bis drei der folgenden Reste tragen können:

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise $C_1$-$C_3$-Alkyl, insbesondere $C_1$-$C_2$-Alkyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise $C_1$-$C_2$-Alkoxy, insbesondere Methoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise $C_1$-$C_2$-Alkylthio;

und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;

steht und

$R^6$ für $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl,

2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_3$-Alkyl, insbesondere $C_1$-$C_2$-Alkyl;

$C_1$-$C_6$-Halogenalkyl, vorzugsweise $C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Chlormethyl, Trifluormethyl und Pentafluorethyl;

Phenyl oder Benzyl steht, wobei die aromatischen Gruppen ihrerseits ein bis fünf Halogenatome wie Fluor, Chlor, Brom und Jod, vorzugsweise Fluor, Chlor und Brom, insbesondere Chlor und Brom;

und/oder ein bis drei der folgenden Reste tragen können:

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise $C_1$-$C_2$-Alkyl, insbesondere Methyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise $C_1$-$C_3$-Alkoxy, insbesondere $C_1$-$C_2$-Alkoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy;

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise $C_1$-$C_2$-Alkylthio, insbesondere Methylthio;

und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio;

sowie deren landwirtschaftlich brauchbaren Salze.

Besonders bevorzugt sind 2-Anilino-cyanopyridine der allgemeinen Formel I, in denen die Reste die folgende Bedeutung haben:

$R^1$ Wasserstoff oder Halogen wie vorstehend genannt, bevorzugt Fluor, Chlor und Brom;

$R^2$ und $R^3$ unabhängig voneinander

Wasserstoff; Nitro; Cyano;

Halogen wie vorstehend genannt, bevorzugt Fluor, Chlor und Brom;

$C_1$-$C_4$-Halogenalkyl wie vorstehend genannt, bevorzugt Trichlormethyl und Trifluormethyl;

$C_1$-$C_4$-Alkoxy wie vorstehend genannt, bevorzugt Methoxy, Ethoxy und Propyloxy;

oder $C_1$-$C_4$-Halogenalkoxy wie vorstehend genannt, bevorzugt Trifluormethoxy;

$R^4$ Wasserstoff; $COR^5$ oder $SO_2R^6$, wobei

$R^5$ für $C_1$-$C_6$-Alkyl wie vorstehend genannt, bevorzugt $C_1$-$C_4$-Alkyl, insbesondere $C_1$-$C_2$-Alkyl;

$C_1$-$C_6$-Halogenalkyl wie vorstehend genannt, bevorzugt $C_1$-$C_2$-Halogenalkyl;

$C_1$-$C_4$-Alkoxy wie vorstehend genannt, bevorzugt $C_1$-$C_2$-Alkoxy;

$C_1$-$C_4$-Halogenalkoxy wie vorstehend genannt, bevorzugt Trifluormethyloxy;

$C_1$-$C_4$-Alkylthio wie vorstehend genannt, bevorzugt $C_1$-$C_2$-Alkylthio;

$C_1$-$C_4$-Halogenalkylthio wie vorstehend genannt, bevorzugt Trifluormethylthio;

$C_1$-$C_4$-Alkylamino wie vorstehend genannt, bevorzugt $C_1$-$C_3$-Alkylamino, insbesondere $C_1$-$C_2$-Alkylamino;

Di-$C_1$-$C_4$-alkylamino wie vorstehend genannt, bevorzugt Di-$C_1$-$C_2$-Alkylamino;
Benzyl, Benzyloxy, Benzylthio oder Benzylamino steht, wobei die aromatischen Gruppen ihrerseits ein bis fünf Halogenatome wie vorstehend genannt, bevorzugt Fluor, Chlor und Brom;
und/oder ein bis drei der folgenden Reste tragen können:
$C_1$-$C_4$-Alkyl wie vorstehend genannt, bevorzugt $C_1$-$C_3$-Alkyl;
$C_1$-$C_4$-Halogenalkyl wie vorstehend genannt, bevorzugt Trifluormethyl;
$C_1$-$C_4$-Alkoxy wie vorstehend genannt, bevorzugt Methoxy und Ethoxy;
$C_1$-$C_4$-Halogenalkoxy wie vorstehend genannt, bevorzugt Trifluormethoxy;
$C_1$-$C_4$-Alkylthio wie vorstehend genannt, bevorzugt $C_1$-$C_2$-Alkylthio;
und $C_1$-$C_4$-Halogenalkylthio wie vorstehend genannt, bevorzugt Trifluormethylthio;
und

$R^6$ Phenyl oder Benzyl steht, wobei die aromatischen Gruppen ihrerseits ein bis fünf Halogenatome wie vorstehend genannt, bevorzugt Fluor, Chlor und Brom;
und/oder ein bis drei der folgenden Reste tragen können:
$C_1$-$C_4$-Alkyl wie vorstehend genannt, bevorzugt $C_1$-$C_2$-Alkyl;
$C_1$-$C_4$-Halogenalkyl wie vorstehend genannt, bevorzugt Trifluormethyl;
$C_1$-$C_4$-Alkoxy wie vorstehend genannt, bevorzugt $C_1$-$C_3$-Alkoxy, insbesondere $C_1$-$C_2$-Alkoxy;
$C_1$-$C_4$-Halogenalkoxy wie vorstehend genannt, bevorzugt Trifluormethoxy;
$C_1$-$C_4$-Alkylthio wie vorstehend genannt, bevorzugt $C_1$-$C_3$-Alkylthio;
und $C_1$-$C_4$-Halogenalkylthio wie vorstehend genannt, bevorzugt Trifluormethylthio;

sowie deren landwirtschaftlich brauchbaren Salze. Insbesondere bevorzugt sind 2-Anilinocyanopyridine der allgemeinen Formel I, in denen die Reste die folgende Bedeutung haben:

$R^1$ Wasserstoff oder Halogen wie vorstehend genannt, bevorzugt Fluor und Chlor;

$R^2$ und $R^3$ unabhängig voneinander Wasserstoff; Nitro; Cyano;
Halogen wie vorstehend genannt, bevorzugt Fluor und Chlor;
oder $C_1$-$C_2$-Halogenalkyl wie vorstehend genannt, bevorzugt Trifluormethyl;

$R^4$ Wasserstoff oder $SO_2R^6$, wobei

$R^6$ Phenyl steht, welches ein bis fünf Halogenatome wie vorstehend genannt, bevorzugt Chlor und Brom;
und/oder ein bis drei der folgenden Reste tragen kann:
$C_1$-$C_4$-Alkyl wie vorstehend genannt, bevorzugt Methyl;
$C_1$-$C_4$-Halogenalkyl wie vorstehend genannt, bevorzugt Trifluormethyl;
$C_1$-$C_4$-Alkoxy wie vorstehend genannt, bevorzugt Methoxy und Ethoxy;
$C_1$-$C_4$-Halogenalkoxy wie vorstehend genannt, bevorzugt Trifluormethoxy;
$C_1$-$C_4$-Alkylthio wie vorstehend genannt, bevorzugt Methylthio;
und $C_1$-$C_4$-Halogenalkylthio wie vorstehend genannt, bevorzugt Trifluormethylthio;
sowie deren landwirtschaftlich brauchbaren Salze.

Als Salze der Verbindungen der Formel I kommen landwirtschaftlich brauchbare Salze, beispielweise Alkalimetallsalze, insbesondere Natrium- und Kaliumsalze, Erdalkalimetallsalze wie insbesondere Calzium-, Magnesium- und Bariumsalze, Mangan-, Kupfer-, Zink- oder Eisensalze sowie Ammoniumsalze wie Tetraalkyl-, Trialkyl-, Dialkyl-, Alkyl- und Benzyltrialkylammoniumsalze, Phosphonium-, Sulfoniumsalze wie Trialkylsulfoniumsalze oder Sulfoxoniumsalze in Betracht.

Beispiele für insbesondere bevorzugte Verbindungen der allgemeinen Formel I sind in den folgenden Tabellen A bis D aufgeführt.

Tabelle A: 3-Cyanopyridinderivate

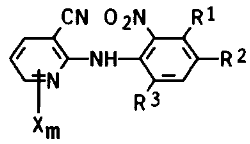

I $R^4$ = H)

| $X_m$ | $R^1$ | $R^2$ | $R^3$ |
|---|---|---|---|
| H | Cl | $CF_3$ | $NO_2$ |
| H | Cl | CN | $NO_2$ |
| H | Cl | $COOC_2H_5$ | $NO_2$ |
| H | Cl | $CONH_2$ | $NO_2$ |
| H | Cl | COOH | $NO_2$ |
| H | F | $CF_3$ | $NO_2$ |
| H | $OCH_3$ | $CF_3$ | $NO_2$ |
| H | H | $CF_3$ | $NO_2$ |
| H | H | CN | $NO_2$ |
| H | H | Cl | $NO_2$ |
| H | Cl | Cl | $NO_2$ |
| H | H | $SO_2CH_3$ | $NO_2$ |
| H | H | $COOCH_3$ | $NO_2$ |
| H | H | $CH_3$ | $NO_2$ |
| H | H | $NO_2$ | $CF_3$ |
| H | H | $NO_2$ | Cl |
| H | H | $NO_2$ | 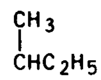 |
| H | Cl | Cl | Cl |
| 5-Cl | Cl | $CF_3$ | $NO_2$ |
| 5-Cl | F | $CF_3$ | $NO_2$ |
| 5-Cl | Br | $CF_3$ | $NO_2$ |
| 5-Cl | $CF_3$ | $CF_3$ | $NO_2$ |
| 5-Cl | $OCH_3$ | $CF_3$ | $NO_2$ |
| 5-Cl | $OC_2H_5$ | $CF_3$ | $NO_2$ |
| 5-Cl | $OC_4H_9$(n) | $CF_3$ | $NO_2$ |
| 5-Cl | $OCF_3$ | $CF_3$ | $NO_2$ |
| 5-Cl | $OCF_2CHF_2$ | $CF_3$ | $NO_2$ |
| 5-Cl | $OC_6H_5$ | $CF_3$ | $NO_2$ |
| 5-Cl | $SC_6H_5$ | $CF_3$ | $NO_2$ |
| 5-Cl | $OC_6H_4$-p-Cl | $CF_3$ | $NO_2$ |
| 5-Cl | $SC_6H_4$-p-Cl | $CF_3$ | $NO_2$ |
| 5-Cl | H | $CF_3$ | $NO_2$ |

Tabelle A (Fortsetzung)

| $X_m$ | $R^1$ | $R^2$ | $R^3$ |
|---|---|---|---|
| 5-Cl | H | H | Cl |
| 5-Cl | H | Br | $NO_2$ |
| 5-Cl | H | Cl | $NO_2$ |
| 5-Cl | H | CN | $NO_2$ |
| 5-Cl | H | $CONH_2$ | $NO_2$ |
| 5-Cl | H | $CON(CH_3)_2$ | $NO_2$ |
| 5-Cl | H | COOH | $NO_2$ |
| 5-Cl | H | $COOCH_3$ | $NO_2$ |
| 5-Cl | Cl | CN | $NO_2$ |
| 5-Cl | Cl | $COOC_2H_5$ | $NO_2$ |
| 5-Cl | Cl | $CONH_2$ | $NO_2$ |
| 5-Cl | Cl | COOH | $NO_2$ |
| 5-Cl | H | $SO_2CH_3$ | $NO_2$ |
| 5-Cl | H | $SO_2NH_2$ | $NO_2$ |
| 5-Cl | H | $SO_2N(CH_3)_2$ | $NO_2$ |
| 5-Cl | H | $CH_3$ | $NO_2$ |
| 5-Cl | H | $C(CH_3)_3$ | $NO_2$ |
| 5-Cl | H | $OCF_3$ | $NO_2$ |
| 5-Cl | H | $OCF_2CHF_2$ | $NO_2$ |
| 5-Cl | H | $NO_2$ | $CF_3$ |
| 5-Cl | H | $NO_2$ | Cl |
| 5-Cl | H | $NO_2$ | $CH_3$ |
| 5-Cl | H | $NO_2$ | $CH(CH_3)C_2H_5$ |
| 5-Cl | Cl | Cl | $NO_2$ |
| 5-Cl | Cl | Cl | Cl |
| 5-Cl | H | $CF_3$ | H |
| 5-Cl | Cl | H | H |
| 5-$CH_3$ | Cl | $CF_3$ | $NO_2$ |
| 5-$CH_3$ | F | $CF_3$ | $NO_2$ |
| 5-$CH_3$ | H | $CF_3$ | $NO_2$ |
| 5-$CH_3$ | H | $NO_2$ | $CF_3$ |
| 5-$CH_3$ | H | $NO_2$ | Cl |
| 5-$CH_3$ | Cl | Cl | $NO_2$ |
| 5-$CH_3$ | Cl | CN | $NO_2$ |
| 5-$CH_3$ | Cl | $COOC_2H_5$ | $NO_2$ |
| 5-Br | Cl | $CF_3$ | $NO_2$ |
| 5-Br | Cl | CN | $NO_2$ |
| 5-Br | Cl | $COOC_2H_5$ | $NO_2$ |
| 5-Br | Cl | $CONH_2$ | $NO_2$ |

Tabelle A (Fortsetzung)

| $X_m$ | $R^1$ | $R^2$ | $R^3$ |
|---|---|---|---|
| 5-Br | F | $CF_3$ | $NO_2$ |
| 5-Br | $OCH_3$ | $CF_3$ | $NO_2$ |
| 5-Br | H | $CF_3$ | $NO_2$ |
| 5-Br | H | CN | $NO_2$ |
| 5-Br | H | Cl | $NO_2$ |
| 5-Br | Cl | Cl | $NO_2$ |
| 5-Br | H | $SO_2CH_3$ | $NO_2$ |
| 5-Br | H | $COOCH_3$ | $NO_2$ |
| 5-Br | H | $CH_3$ | $NO_2$ |
| 5-Br | H | $NO_2$ | $CF_3$ |
| 5-Br | H | $NO_2$ | Cl |
| 5-Br | H | $NO_2$ | $CH_3$<br>$CH-C_2H_5$ |
| 5-Br | Cl | Cl | Cl |
| 5-$CF_3$ | Cl | $CF_3$ | $NO_2$ |
| 5-$CF_3$ | F | $CF_3$ | $NO_2$ |
| 5-$CF_3$ | H | $CF_3$ | $NO_2$ |
| 5-$CF_3$ | H | $NO_2$ | $CF_3$ |
| 5-$CF_3$ | H | $NO_2$ | Cl |
| 5-$CF_3$ | Cl | Cl | $NO_2$ |
| 5-$CF_3$ | Cl | CN | $NO_2$ |
| 5-$CF_3$ | Cl | $COOC_2H_5$ | $NO_2$ |
| 6-Cl | Cl | $CF_3$ | $NO_2$ |
| 6-Cl | F | $CF_3$ | $NO_2$ |
| 6-Cl | Br | $CF_3$ | $NO_2$ |
| 6-Cl | $CF_3$ | $CF_3$ | $NO_2$ |
| 6-Cl | $OCH_3$ | $CF_3$ | $NO_2$ |
| 6-Cl | $OC_2H_5$ | $CF_3$ | $NO_2$ |
| 6-Cl | O-n-$C_4H_9$ | $CF_3$ | $NO_2$ |
| 6-Cl | $OCF_3$ | $CF_3$ | $NO_2$ |
| 6-Cl | $OCF_2CHF_2$ | $CF_3$ | $NO_2$ |
| 6-Cl | $OC_6H_5$ | $CF_3$ | $NO_2$ |
| 6-Cl | $SC_6H_5$ | $CF_3$ | $NO_2$ |
| 6-Cl | $OC_6H_4$-p-Cl | $CF_3$ | $NO_2$ |
| 6-Cl | $SC_6H_4$-p-Cl | $CF_3$ | $NO_2$ |
| 6-Cl | H | $CF_3$ | $NO_2$ |
| 6-Cl | H | H | Cl |
| 6-Cl | H | Br | $NO_2$ |
| 6-Cl | H | Cl | $NO_2$ |
| 6-Cl | H | CN | $NO_2$ |

Tabelle A (Fortsetzung)

| $X_m$ | $R^1$ | $R^2$ | $R^3$ |
|---|---|---|---|
| 6-Cl | H | $CONH_2$ | $NO_2$ |
| 6-Cl | H | $CON(CH_3)_2$ | $NO_2$ |
| 6-Cl | H | COOH | $NO_2$ |
| 6-Cl | H | $COOCH_3$ | $NO_2$ |
| 6-Cl | Cl | CN | $NO_2$ |
| 6-Cl | Cl | $COOC_2H_5$ | $NO_2$ |
| 6-Cl | Cl | $CONH_2$ | $NO_2$ |
| 6-Cl | Cl | COOH | $NO_2$ |
| 6-Cl | H | $SO_2CH_3$ | $NO_2$ |
| 6-Cl | H | $SO_2NH_2$ | $NO_2$ |
| 6-Cl | H | $SO_2N(CH_3)_2$ | $NO_2$ |
| 6-Cl | H | $CH_3$ | $NO_2$ |
| 6-Cl | H | $C(CH_3)_3$ | $NO_2$ |
| 6-Cl | H | $OCF_3$ | $NO_2$ |
| 6-Cl | H | $OCF_2CHF_2$ | $NO_2$ |
| 6-Cl | H | $NO_2$ | $CF_3$ |
| 6-Cl | H | $NO_2$ | Cl |
| 6-Cl | H | $NO_2$ | $CH_3$ |
| 6-Cl | H | $NO_2$ | $\begin{matrix}CH_3\\ \vert\\ CH-C_2H_5\end{matrix}$ |
| 6-Cl | Cl | Cl | $NO_2$ |
| 6-Cl | Cl | Cl | Cl |
| 6-Cl | H | $CF_3$ | H |
| 6-Br | Cl | $CF_3$ | $NO_2$ |
| 6-Br | F | $CF_3$ | $NO_2$ |
| 6-Br | H | $CF_3$ | $NO_2$ |
| 6-Br | H | $NO_2$ | $CF_3$ |
| 6-Br | H | $NO_2$ | Cl |
| 6-Br | Cl | Cl | $NO_2$ |
| 6-Br | Cl | CN | $NO_2$ |
| 6-Br | Cl | $COOC_2H_5$ | $NO_2$ |
| 6-cyclo-$C_3H_5$ | Cl | $CF_3$ | $NO_2$ |
| 6-cyclo-$C_3H_5$ | F | $CF_3$ | $NO_2$ |
| 6-cyclo-$C_3H_5$ | H | $CF_3$ | $NO_2$ |
| 6-cyclo-$C_3H_5$ | H | $NO_2$ | $CF_3$ |
| 6-cyclo-$C_3H_5$ | H | $NO_2$ | Cl |
| 6-cyclo-$C_3H_5$ | Cl | Cl | $NO_2$ |
| 6-cyclo-$C_3H_5$ | Cl | CN | $NO_2$ |
| 6-cyclo-$C_3H_5$ | Cl | $COOC_2H_5$ | $NO_2$ |
| 6-$CH(CH_3)_2$ | Cl | $CF_3$ | $NO_2$ |

Tabelle A (Fortsetzung)

| $X_m$ | $R^1$ | $R^2$ | $R^3$ |
|---|---|---|---|
| 6-$CH(CH_3)_2$ | F | $CF_3$ | $NO_2$ |
| 6-$CH(CH_3)_2$ | H | $CF_3$ | $NO_2$ |
| 6-$CH(CH_3)_2$ | H | $NO_2$ | $CF_3$ |
| 6-$CH(CH_3)_2$ | H | $NO_2$ | Cl |
| 6-$CH(CH_3)_2$ | Cl | Cl | $NO_2$ |
| 6-$CH(CH_3)_2$ | Cl | CN | $NO_2$ |
| 6-$CH(CH_3)_2$ | Cl | $COOC_2H_5$ | $NO_2$ |
| 6-$CH_3$ | Cl | $CF_3$ | $NO_2$ |
| 6-$CH_3$ | F | $CF_3$ | $NO_2$ |
| 6-$CH_3$ | H | $CF_3$ | $NO_2$ |
| 6-$CH_3$ | H | $NO_2$ | $CF_3$ |
| 6-$CH_3$ | H | $NO_2$ | Cl |
| 6-$CH_3$ | Cl | Cl | $NO_2$ |
| 6-$CH_3$ | Cl | CN | $NO_2$ |
| 6-$CH_3$ | Cl | $COOC_2H_5$ | $NO_2$ |
| 6-CN | Cl | $CF_3$ | $NO_2$ |
| 6-CN | F | $CF_3$ | $NO_2$ |
| 6-CN | H | $CF_3$ | $NO_2$ |
| 6-CN | H | $NO_2$ | $CF_3$ |
| 6-CN | H | $NO_2$ | Cl |
| 6-CN | Cl | Cl | $NO_2$ |
| 6-CN | Cl | CN | $NO_2$ |
| 6-CN | Cl | $COOC_2H_5$ | $NO_2$ |
| 6-$SC_6H_5$ | Cl | $CF_3$ | $NO_2$ |
| 6-$SC_6H_5$ | F | $CF_3$ | $NO_2$ |
| 6-$SC_6H_5$ | H | $CF_3$ | $NO_2$ |
| 6-$SC_6H_5$ | H | $NO_2$ | $CF_3$ |
| 6-$SC_6H_5$ | H | $NO_2$ | Cl |
| 6-$SC_6H_5$ | Cl | Cl | $NO_2$ |
| 6-$SC_6H_5$ | Cl | CN | $NO_2$ |
| 6-$SC_6H_5$ | Cl | $COOC_2H_5$ | $NO_2$ |
| 6-$SC_6H_5$ | Cl | $CF_3$ | $NO_2$ |
| 6-$SC_6H_5$ | F | $CF_3$ | $NO_2$ |
| 6-$SC_6H_5$ | H | $CF_3$ | $NO_2$ |
| 6-$SC_6H_5$ | H | $NO_2$ | $CF_3$ |
| 6-$SC_6H_5$ | H | $NO_2$ | Cl |
| 6-$SC_6H_5$ | Cl | Cl | $NO_2$ |
| 6-$SC_6H_5$ | Cl | CN | $NO_2$ |
| 6-$SC_6H_5$ | Cl | $COOC_2H_5$ | $NO_2$ |
| 6-$OC_2H_5$ | Cl | $CF_3$ | $NO_2$ |

Tabelle A (Fortsetzung)

| $X_m$ | $R^1$ | $R^2$ | $R^3$ |
|---|---|---|---|
| 6-$OC_2H_5$ | F | $CF_3$ | $NO_2$ |
| 6-$OC_2H_5$ | H | $CF_3$ | $NO_2$ |
| 6-$OC_2H_5$ | H | $NO_2$ | $CF_3$ |
| 6-$OC_2H_5$ | H | $NO_2$ | Cl |
| 6-$OC_2H_5$ | Cl | Cl | $NO_2$ |
| 6-$OC_2H_5$ | Cl | CN | $NO_2$ |
| 6-$OC_2H_5$ | Cl | $COOC_2H_5$ | $NO_2$ |
| 4,6-$(CH_3)_2$ | Cl | $CF_3$ | $NO_2$ |
| 4,6-$(CH_3)_2$ | F | $CF_3$ | $NO_2$ |
| 4,6-$(CH_3)_2$ | H | $CF_3$ | $NO_2$ |
| 4,6-$(CH_3)_2$ | H | $NO_2$ | $CF_3$ |
| 4,6-$(CH_3)_2$ | H | $NO_2$ | Cl |
| 4,6-$(CH_3)_2$ | H | $C(CH_3)_3$ | $NO_2$ |
| 4,6-$(CH_3)_2$ | Cl | Cl | $NO_2$ |
| 4,6-$(CH_3)_2$ | Cl | CN | $NO_2$ |
| 4,6-$(CH_3)_2$ | H | CN | $NO_2$ |
| 4,6-$(CH_3)_2$ | Cl | $COOC_2H_5$ | $NO_2$ |
| 4-$CH_3$, 6-Cl | Cl | $CF_3$ | $NO_2$ |
| 4-$CH_3$, 6-Cl | F | $CF_3$ | $NO_2$ |
| 4-$CH_3$, 6-Cl | H | $CF_3$ | $NO_2$ |
| 4-$CH_3$, 6-Cl | H | $NO_2$ | $CF_3$ |
| 4-$CH_3$, 6-Cl | H | $NO_2$ | Cl |
| 4-$CH_3$, 6-Cl | Cl | Cl | $NO_2$ |
| 4-$CH_3$, 6-Cl | Cl | CN | $NO_2$ |
| 4-$CH_3$, 6-Cl | Cl | $COOC_2H_5$ | $NO_2$ |
| 5-CN | Cl | $CF_3$ | $NO_2$ |
| 5-CN | F | $CF_3$ | $NO_2$ |
| 5-CN | H | $CF_3$ | $NO_2$ |
| 5-CN | H | $NO_2$ | $CF_3$ |
| 5-CN | H | $NO_2$ | Cl |
| 5-CN | Cl | Cl | $NO_2$ |
| 5-CN | Cl | CN | $NO_2$ |
| 5-CN | Cl | $COOC_2H_5$ | $NO_2$ |
| 5-CN, 6-$CH_3$ | Cl | $CF_3$ | $NO_2$ |
| 5-CN, 6-$CH_3$ | F | $CF_3$ | $NO_2$ |
| 5-CN, 6-$CH_3$ | H | $CF_3$ | $NO_2$ |
| 5-CN, 6-$CH_3$ | H | $NO_2$ | $CF_3$ |
| 5-CN, 6-$CH_3$ | H | $NO_2$ | Cl |
| 5-CN, 6-$CH_3$ | Cl | Cl | $NO_2$ |
| 5-CN, 6-$CH_3$ | Cl | CN | $NO_2$ |

Tabelle A (Fortsetzung)

| $X_m$ | $R^1$ | $R^2$ | $R^3$ |
|---|---|---|---|
| 5-CN, 6-$CH_3$ | Cl | $COOC_2H_5$ | $NO_2$ |
| 4-$CH_3$, 5-CN, 6-$OC_2H_5$ | Cl | $CF_3$ | $NO_2$ |
| 4-$CH_3$, 5-CN, 6-$OC_2H_5$ | F | $CF_3$ | $NO_2$ |
| 4-$CH_3$, 5-CN, 6-$OC_2H_5$ | H | $CF_3$ | $NO_2$ |
| 4-$CH_3$, 5-CN, 6-$OC_2H_5$ | H | $NO_2$ | $CF_3$ |
| 4-$CH_3$, 5-CN, 6-$OC_2H_5$ | H | $NO_2$ | Cl |
| 4-$CH_3$, 5-CN, 6-$OC_2H_5$ | Cl | Cl | $NO_2$ |
| 4-$CH_3$, 5-CN, 6-$OC_2H_5$ | Cl | CN | $NO_2$ |
| 4-$CH_3$, 5-CN, 6-$OC_2H_5$ | Cl | $COOC_2H_5$ | $NO_2$ |
| 4-$CH(CH_3)_2$, 5-CN, 6-$OC_2H_5$ | Cl | $CF_3$ | $NO_2$ |
| 4-$CH(CH_3)_2$, 5-CN, 6-$OC_2H_5$ | Cl | CN | $NO_2$ |
| 4-$CH(CH_3)_2$, 5-CN, 6-$OC_2H_5$ | Cl | $COOC_2H_5$ | $NO_2$ |
| 4-$CH(CH_3)_2$, 5-CN, 6-$OC_2H_5$ | Cl | $CONH_2$ | $NO_2$ |
| 4-$CH(CH_3)_2$, 5-CN, 6-$OC_2H_5$ | Cl | COOH | $NO_2$ |
| 4-$CH(CH_3)_2$, 5-CN, 6-$OC_2H_5$ | F | $CF_3$ | $NO_2$ |
| 4-$CH(CH_3)_2$, 5-CN, 6-$OC_2H_5$ | $OCH_3$ | $CF_3$ | $NO_2$ |
| 4-$CH(CH_3)_2$, 5-CN, 6-$OC_2H_5$ | H | $CF_3$ | $NO_2$ |
| 4-$CH(CH_3)_2$, 5-CN, 6-$OC_2H_5$ | H | CN | $NO_2$ |
| 4-$CH(CH_3)_2$, 5-CN, 6-$OC_2H_5$ | H | Cl | $NO_2$ |
| 4-$CH(CH_3)_2$, 5-CN, 6-$OC_2H_5$ | Cl | Cl | $NO_2$ |
| 4-$CH(CH_3)_2$, 5-CN, 6-$OC_2H_5$ | H | $SO_2CH_3$ | $NO_2$ |
| 4-$CH(CH_3)_2$, 5-CN, 6-$OC_2H_5$ | H | $COOCH_3$ | $NO_2$ |
| 4-$CH(CH_3)_2$, 5-CN, 6-$OC_2H_5$ | H | $CH_3$ | $NO_2$ |
| 4-$CH(CH_3)_2$, 5-CN, 6-$OC_2H_5$ | H | $NO_2$ | $CF_3$ |
| 4-$CH(CH_3)_2$, 5-CN, 6-$OC_2H_5$ | H | $NO_2$ | Cl |
| 4-$CH(CH_3)_2$, 5-CN, 6-$OC_2H_5$ | H | $NO_2$ | $CH_3$<br>$CH-C_2H_5$ |
| 4-$CH(CH_3)_2$, 5-CN, 6-$OC_2H_5$ | Cl | Cl | Cl |
| 5-$NO_2$ | Cl | $CF_3$ | $NO_2$ |
| 5-$NO_2$ | F | $CF_3$ | $NO_2$ |
| 5-$NO_2$ | H | $CF_3$ | $NO_2$ |
| 5-$NO_2$ | H | $NO_2$ | $CF_3$ |
| 5-$NO_2$ | H | $NO_2$ | Cl |
| 5-$NO_2$ | Cl | Cl | $NO_2$ |
| 5-$NO_2$ | Cl | CN | $NO_2$ |
| 5-$NO_2$ | Cl | $COOC_2H_5$ | $NO_2$ |
| 6-$SO_2CH_3$ | Cl | $CF_3$ | $NO_2$ |
| 6-$SO_2CH_3$ | H | $NO_2$ | $CF_3$ |
| 6-$OCH_3$ | Cl | $CF_3$ | $NO_2$ |

Tabelle A (Fortsetzung)

| $X_m$ | $R^1$ | $R^2$ | $R^3$ |
|---|---|---|---|
| 6-$OCH_3$ | H | $NO_2$ | $CF_3$ |
| 4-$CH_3$, 5-CN, 6-$OCH_3$ | Cl | $CF_3$ | $NO_2$ |
| 4-$CH_3$, 5-CN, 6-$OCH_3$ | H | $NO_2$ | $CF_3$ |
| 4-$CH(CH_3)_2$, 5-CN, 6-$OCH_3$ | Cl | $CF_3$ | $NO_2$ |
| 4-$CH(CH_3)_2$, 5-CN, 6-$OCH_3$ | H | $NO_2$ | $CF_3$ |
| 4-$C_2H_5$, 5-CN, 6-$OC_2H_5$ | Cl | $CF_3$ | $NO_2$ |
| 4-$C_2H_5$, 5-CN, 6-$OC_2H_5$ | H | $NO_2$ | $CF_3$ |
| 4-$CH_3$, 6-Br | Cl | $CF_3$ | $NO_2$ |
| 4-$CH_3$, 6-Br | H | $NO_2$ | $CF_3$ |
| 6-cyclo-$C_5H_9$ | Cl | $CF_3$ | $NO_2$ |
| 6-cyclo-$C_5H_9$ | H | $NO_2$ | $CF_3$ |
| 4-cyclo-$C_3H_5$, 6-Cl | Cl | $CF_3$ | $NO_2$ |
| 4-cyclo-$C_3H_5$, 6-Cl | H | $NO_2$ | $CF_3$ |
| 5-CN, 6-cyclo-$C_3H_5$ | Cl | $CF_3$ | $NO_2$ |
| 5-CN, 6-cyclo-$C_3H_5$ | H | $NO_2$ | $CF_3$ |
| 6-OPh-4-Cl | Cl | $CF_3$ | $NO_2$ |
| 6-OPh-4-Cl | H | $NO_2$ | $CF_3$ |
| 6-OPh-4-$CH_3$ | Cl | $CF_3$ | $NO_2$ |
| 6-OPh-4-$CH_3$ | H | $NO_2$ | $CF_3$ |
| 6-SPh-4-$CH_3$ | Cl | $CF_3$ | $NO_2$ |
| 6-SPh-4-$CH_3$ | H | $NO_2$ | $CF_3$ |
| 5-Cl,6-$CH_3$ | H | $CF_3$ | $NO_2$ |
| 5-Cl,6-$CH_3$ | Cl | $CF_3$ | $NO_2$ |
| 5-Cl,6-$CH_3$ | H | $NO_2$ | $CF_3$ |
| 5-Cl,6-$CH_3$ | H | $NO_2$ | Cl |
| 5-Br,6-$CH_3$ | H | $CF_3$ | $NO_2$ |
| 5-Br,6-$CH_3$ | Cl | $CF_3$ | $NO_2$ |
| 5-Br,6-$CH_3$ | H | $NO_2$ | $CF_3$ |
| 5-Br,6-$CH_3$ | H | $NO_2$ | Cl |
| 5-Cl,4,6-$(CH_3)_2$ | H | $CF_3$ | $NO_2$ |
| 5-Cl,4,6-$(CH_3)_2$ | Cl | $CF_3$ | $NO_2$ |
| 5-Cl,4,6-$(CH_3)_2$ | H | $NO_2$ | $CF_3$ |
| 5-Cl,4,6-$(CH_3)_2$ | H | $NO_2$ | Cl |
| 5-Br,4,6-$(CH_3)_2$ | H | $CF_3$ | $NO_2$ |
| 5-Br,4,6-$(CH_3)_2$ | Cl | $CF_3$ | $NO_2$ |
| 5-Br,4,6-$(CH_3)_2$ | H | $NO_2$ | $CF_3$ |
| 5-Br,4,6-$(CH_3)_2$ | H | $NO_2$ | Cl |

Tabelle B: 5-Cyanopyridinderivate

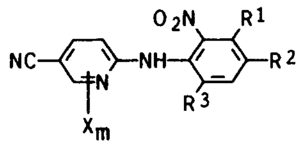

I $R^4$ = H)

| $X_m$ | $R^1$ | $R^2$ | $R^3$ |
|---|---|---|---|
| H | Cl | $CF_3$ | $NO_2$ |
| H | Cl | CN | $NO_2$ |
| H | Cl | $COOC_2H_5$ | $NO_2$ |
| H | Cl | $CONH_2$ | $NO_2$ |
| H | Cl | COOH | $NO_2$ |
| H | F | $CF_3$ | $NO_2$ |
| H | $OCH_3$ | $CF_3$ | $NO_2$ |
| H | H | $CF_3$ | $NO_2$ |
| H | H | CN | $NO_2$ |
| H | H | Cl | $NO_2$ |
| H | Cl | Cl | $NO_2$ |
| H | H | $SO_2CH_3$ | $NO_2$ |
| H | H | $COOCH_3$ | $NO_2$ |
| H | H | $CH_3$ | $NO_2$ |
| H | H | $NO_2$ | $CF_3$ |
| H | H | $NO_2$ | Cl |
| H | H | $NO_2$ | $CH_3$<br>\|<br>$CH-C_2H_5$ |
| H | Cl | Cl | Cl |
| 3-Cl | Cl | $CF_3$ | $NO_2$ |
| 3-Cl | F | $CF_3$ | $NO_2$ |
| 3-Cl | Br | $CF_3$ | $NO_2$ |
| 3-Cl | $CF_3$ | $CF_3$ | $NO_2$ |
| 3-Cl | $OCH_3$ | $CF_3$ | $NO_2$ |
| 3-Cl | $OC_2H_5$ | $CF_3$ | $NO_2$ |
| 3-Cl | $O-n-C_4H_9$ | $CF_3$ | $NO_2$ |
| 3-Cl | $OCF_3$ | $CF_3$ | $NO_2$ |
| 3-Cl | $OCF_2CHF_2$ | $CF_3$ | $NO_2$ |
| 3-Cl | $OC_6H_5$ | $CF_3$ | $NO_2$ |
| 3-Cl | $SC_6H_5$ | $CF_3$ | $NO_2$ |
| 3-Cl | $OC_6H_4$-p-Cl | $CF_3$ | $NO_2$ |
| 3-Cl | $SC_6H_4$-p-Cl | $CF_3$ | $NO_2$ |
| 3-Cl | H | $CF_3$ | $NO_2$ |
| 3-Cl | H | H | Cl |

Tabelle B (Fortsetzung)

| $X_m$ | $R^1$ | $R^2$ | $R^3$ |
|---|---|---|---|
| 3-Cl | H | Br | $NO_2$ |
| 3-Cl | H | Cl | $NO_2$ |
| 3-Cl | H | CN | $NO_2$ |
| 3-Cl | H | $CONH_2$ | $NO_2$ |
| 3-Cl | H | $CON(CH_3)_2$ | $NO_2$ |
| 3-Cl | H | COOH | $NO_2$ |
| 3-Cl | H | $COOCH_3$ | $NO_2$ |
| 3-Cl | Cl | CN | $NO_2$ |
| 3-Cl | Cl | $COOC_2H_5$ | $NO_2$ |
| 3-Cl | Cl | $CONH_2$ | $NO_2$ |
| 3-Cl | Cl | COOH | $NO_2$ |
| 3-Cl | H | $SO_2CH_3$ | $NO_2$ |
| 3-Cl | H | $SO_2NH_2$ | $NO_2$ |
| 3-Cl | H | $SO_2N(CH_3)_2$ | $NO_2$ |
| 3-Cl | H | $CH_3$ | $NO_2$ |
| 3-Cl | H | $C(CH_3)_3$ | $NO_2$ |
| 3-Cl | H | $OCF_3$ | $NO_2$ |
| 3-Cl | H | $OCF_2CHF_2$ | $NO_2$ |
| 3-Cl | H | $NO_2$ | $CF_3$ |
| 3-Cl | H | $NO_2$ | Cl |
| 3-Cl | H | $NO_2$ | $CH_3$ |
| 3-Cl | H | $NO_2$ | 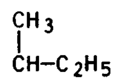 |
| 3-Cl | Cl | Cl | $NO_2$ |
| 3-Cl | Cl | Cl | Cl |
| 3-Cl | H | $CF_3$ | H |
| 3-Cl | Cl | H | H |
| 3-$NO_2$ | Cl | $CF_3$ | $NO_2$ |
| 3-$NO_2$ | F | $CF_3$ | $NO_2$ |
| 3-$NO_2$ | H | $CF_3$ | $NO_2$ |
| 3-$NO_2$ | H | $NO_2$ | $CF_3$ |
| 3-$NO_2$ | H | $NO_2$ | Cl |
| 3-$NO_2$ | Cl | Cl | $NO_2$ |
| 3-$NO_2$ | Cl | CN | $NO_2$ |
| 3-$NO_2$ | Cl | $COOC_2H_5$ | $NO_2$ |
| 3-Br | Cl | $CF_3$ | $NO_2$ |
| 3-Br | Cl | CN | $NO_2$ |
| 3-Br | Cl | $COOC_2H_5$ | $NO_2$ |
| 3-Br | Cl | $CONH_2$ | $NO_2$ |

| $X_m$ | $R^1$ | $R^2$ | $R^3$ |
|---|---|---|---|
| 3-Br | F | $CF_3$ | $NO_2$ |
| 3-Br | $OCH_3$ | $CF_3$ | $NO_2$ |
| 3-Br | H | $CF_3$ | $NO_2$ |
| 3-Br | H | CN | $NO_2$ |
| 3-Br | H | Cl | $NO_2$ |
| 3-Br | Cl | Cl | $NO_2$ |
| 3-Br | H | $SO_2CH_3$ | $NO_2$ |
| 3-Br | H | $COOCH_3$ | $NO_2$ |
| 3-Br | H | $CH_3$ | $NO_2$ |
| 3-Br | H | $NO_2$ | $CF_3$ |
| 3-Br | H | $NO_2$ | Cl |
| 3-Br | H | $NO_2$ | $CH(CH_3)-C_2H_5$ |
| 3-Br | Cl | Cl | Cl |
| $3-CF_3$ | Cl | $CF_3$ | $NO_2$ |
| $3-CF_3$ | F | $CF_3$ | $NO_2$ |
| $3-CF_3$ | H | $CF_3$ | $NO_2$ |
| $3-CF_3$ | H | $NO_2$ | $CF_3$ |
| $3-CF_3$ | H | $NO_2$ | Cl |
| $3-CF_3$ | Cl | Cl | $NO_2$ |
| $3-CF_3$ | Cl | CN | $NO_2$ |
| $3-CF_3$ | Cl | $COOC_2H_5$ | $NO_2$ |
| $4,6-(CH_3)_2$ | Cl | $CF_3$ | $NO_2$ |
| $4,6-(CH_3)_2$ | F | $CF_3$ | $NO_2$ |
| $4,6-(CH_3)_2$ | H | $CF_3$ | $NO_2$ |
| $4,6-(CH_3)_2$ | H | $NO_2$ | $CF_3$ |
| $4,6-(CH_3)_2$ | H | $NO_2$ | Cl |
| $4,6-(CH_3)_2$ | Cl | Cl | $NO_2$ |
| $4,6-(CH_3)_2$ | Cl | CN | $NO_2$ |
| $4,6-(CH_3)_2$ | Cl | $COOC_2H_5$ | $NO_2$ |
| 6-Cl | Cl | $CF_3$ | $NO_2$ |
| 6-Cl | F | $CF_3$ | $NO_2$ |
| 6-Cl | H | $CF_3$ | $NO_2$ |
| 6-Cl | H | $NO_2$ | $CF_3$ |
| 6-Cl | H | $NO_2$ | Cl |
| 6-Cl | Cl | Cl | $NO_2$ |
| 6-Cl | Cl | CN | $NO_2$ |
| 6-Cl | Cl | $COOC_2H_5$ | $NO_2$ |
| $3,6-Cl_2$ | Cl | $CF_3$ | $NO_2$ |
| $3,6-Cl_2$ | F | $CF_3$ | $NO_2$ |

| $X_m$ | $R^1$ | $R^2$ | $R^3$ |
|---|---|---|---|
| 3,6-$Cl_2$ | H | $CF_3$ | $NO_2$ |
| 3,6-$Cl_2$ | H | $NO_2$ | $CF_3$ |
| 3,6-$Cl_2$ | H | $NO_2$ | Cl |
| 3,6-$Cl_2$ | Cl | Cl | $NO_2$ |
| 3,6-$Cl_2$ | Cl | CN | $NO_2$ |
| 3,6-$Cl_2$ | Cl | $COOC_2H_5$ | $NO_2$ |
| 4-$CH_3$ | Cl | $CF_3$ | $NO_2$ |
| 4-$CH_3$ | H | $NO_2$ | $CF_3$ |
| 6-$CH_3$ | Cl | $CF_3$ | $NO_2$ |
| 6-$CH_3$ | H | $NO_2$ | $CF_3$ |
| 3-Cl, 6-$OC_2H_5$ | Cl | $CF_3$ | $NO_2$ |
| 3-Cl, 6-$OC_2H_5$ | H | $NO_2$ | $CF_3$ |
| 3-Cl, 6-$OC_6H_5$ | Cl | $CF_3$ | $NO_2$ |
| 3-Cl, 6-$OC_6H_5$ | H | $NO_2$ | $CF_3$ |
| 3-Cl, 6-$SO_2CH_3$ | Cl | $CF_3$ | $NO_2$ |
| 3-Cl, 6-$SO_2CH_3$ | H | $NO_2$ | $CF_3$ |
| 3-Cl, 6-$CH_3$ | H | $CF_3$ | $NO_2$ |
| 3-Cl, 6-$CH_3$ | Cl | $CF_3$ | $NO_2$ |
| 3-Cl, 6-$CH_3$ | H | $NO_2$ | $CF_3$ |
| 3-Cl, 6-$CH_3$ | H | $NO_2$ | Cl |
| 3-Br, 6-$CH_3$ | H | $CF_3$ | $NO_2$ |
| 3-Br, 6-$CH_3$ | Cl | $CF_3$ | $NO_2$ |
| 3-Br, 6-$CH_3$ | H | $NO_2$ | $CF_3$ |
| 3-Br, 6-$CH_3$ | H | $NO_2$ | Cl |
| 3-Cl, 4,6-$(CH_3)_2$ | H | $CF_3$ | $NO_2$ |
| 3-Cl, 4,6-$(CH_3)_2$ | Cl | $CF_3$ | $NO_2$ |
| 3-Cl, 4,6-$(CH_3)_2$ | H | $NO_2$ | $CF_3$ |
| 3-Cl, 4,6-$(CH_3)_2$ | H | $NO_2$ | Cl |
| 3-Br, 4,6-$(CH_3)_2$ | H | $CF_3$ | $NO_2$ |
| 3-Br, 4,6-$(CH_3)_2$ | Cl | $CF_3$ | $NO_2$ |
| 3-Br, 4,6-$(CH_3)_2$ | H | $NO_2$ | $CF_3$ |
| 3-Br, 4,6-$(CH_3)_2$ | H | $NO_2$ | Cl |

Tabelle C:

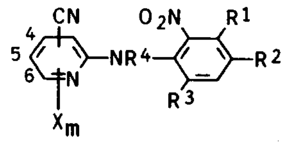

| $X_m$/CN | $R^1$ | $R^2$ | $R^3$ | $R^4$ |
|---|---|---|---|---|
| 3-CN | Cl | $CF_3$ | $NO_2$ | $SO_2C_6H_5$ |
| 3-CN | Cl | $CF_3$ | $NO_2$ | $CH_2-CH=CH_2$ |
| 3-CN | Cl | $CF_3$ | $NO_2$ | $CH_2-C\equiv CH$ |
| 3-CN | H | $NO_2$ | $CF_3$ | $COCH_2Cl$ |
| 5-CN | Cl | $CF_3$ | $NO_2$ | $SO_2C_6H_5$ |
| 5-CN | Cl | $CF_3$ | $NO_2$ | $CONH_2$ |
| 5-CN | H | $NO_2$ | $CF_3$ | $COOCH_2C_6H_5$ |
| 5-CN | H | $NO_2$ | $CF_3$ | $SO_2C_6H_5$ |
| 3-Cl, 5-CN | H | H | Cl | $SO_2C_6H_5$ |
| 3-Cl, 5-CN | Cl | $CF_3$ | $NO_2$ | $CH_2-CH=CH_2$ |
| 3-Cl, 5-CN | Cl | $CF_3$ | $NO_2$ | $CH_2-C\equiv CH$ |
| 3-Cl, 5-CN | H | $CF_3$ | H | $SO_2C_6H_5$ |
| 3-Cl, 5-CN | Cl | $CF_3$ | $NO_2$ | $SO_2C_6H_5$ |
| 3-Cl, 5-CN | Cl | $CF_3$ | $NO_2$ | $COCH_3$ |
| 3-Cl, 5-CN | Cl | $CF_3$ | $NO_2$ | $COCHCl_2$ |
| 3-Cl, 5-CN | Cl | $CF_3$ | $NO_2$ | $COC_6H_5$ |
| 3-Cl, 5-CN | Cl | $CF_3$ | $NO_2$ | $COOCH_3$ |
| 3-Cl, 5-CN | Cl | $CF_3$ | $NO_2$ | $CONH_2$ |
| 3-Cl, 5-CN | Cl | $CF_3$ | $NO_2$ | CHO |
| 3-Cl, 5-CN | H | $NO_2$ | $CF_3$ | $SO_2C_6H_5$ |
| 3-Cl, 5-CN | H | $NO_2$ | $CF_3$ | $COOCH_3$ |
| 3-Cl, 5-CN | H | $NO_2$ | $CF_3$ | $COCH_2Cl$ |
| 6-Cl, 3-CN | Cl | $CF_3$ | $NO_2$ | $SO_2C_6H_5$ |
| 6-Cl, 3-CN | H | $NO_2$ | $CF_3$ | $SO_2C_6H_5$ |
| 6-Cl, 5-CN | Cl | $CF_3$ | $NO_2$ | $SO_2C_6H_5$ |
| 6-Cl, 5-CN | H | $NO_2$ | $CF_3$ | $SO_2C_6H_5$ |
| $4-CH(CH_3)_2$, $6-OC_2H_5$, $3,5-(CN)_2$ | Cl | $CF_3$ | $NO_2$ | $SO_2C_6H_5$ |
| $4-CH(CH_3)_2$, $6-OC_2H_5$, $3,5-(CN)_2$ | H | $NO_2$ | $CF_3$ | $SO_2C_6H_5$ |
| 5-Cl, 3-CN | Cl | $CF_3$ | $NO_2$ | $SO_2C_6H_5$ |
| 5-Cl, 3-CN | Cl | $CF_3$ | $NO_2$ | $COOCH_3$ |
| 5-Cl, 3-CN | Cl | $CF_3$ | $NO_2$ | $CONH_2$ |
| 5-Cl, 3-CN | Cl | $CF_3$ | $NO_2$ | $COC_6H_5$ |

Tabelle C (Fortsetzung)

| $X_m$/CN | $R^1$ | $R^2$ | $R^3$ | $R^4$ |
|---|---|---|---|---|
| 5-Cl, 3-CN | H | $NO_2$ | $CF_3$ | $SO_2C_6H_5$ |
| 5-Cl, 3-CN | H | $NO_2$ | $CF_3$ | $COOCH_3$ |
| 5-Cl, 3-CN | H | $NO_2$ | $CF_3$ | $CONH_2$ |
| 5-Cl, 3-CN | H | $NO_2$ | $CF_3$ | $COC_6H_5$ |
| 5-Cl, 3-CN | H | $NO_2$ | $CF_3$ | $COCHCl_2$ |
| 6-cyclo-$C_3H_5$, 3-CN | Cl | $CF_3$ | $NO_2$ | $SO_2C_6H_5$ |
| 6-$CH_3$, 3,5-$(CN)_2$ | Cl | $CF_3$ | $NO_2$ | $SO_2C_6H_5$ |
| 6-$CH_3$, 3,5-$(CN)_2$ | H | $NO_2$ | $CF_3$ | $SO_2C_6H_5$ |
| 6-Cl, 4-CN | Cl | $CF_3$ | $NO_2$ | $SO_2C_6H_5$ |
| 6-Cl, 4-CN | H | $NO_2$ | $CF_3$ | $SO_2C_6H_5$ |
| 4,6-$(CH_3)_2$, 3-CN | Cl | $CF_3$ | $NO_2$ | $SO_2C_6H_5$ |
| 4,6-$(CH_3)_2$, 3-CN | H | $NO_2$ | $CF_3$ | $SO_2C_6H_5$ |
| 4,6-$(CH_3)_2$, 3-CN | H | $CH_3$ | $NO_2$ | $SO_2C_6H_5$ |

Tabelle D:

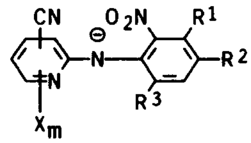

$M^{\oplus}$

| $X_m$/CN | $R^1$ | $R^2$ | $R^3$ | $M^+$ |
|---|---|---|---|---|
| 3-CN | Cl | $CF_3$ | $NO_2$ | $K^+$ |
| 3-CN | Cl | $CF_3$ | $NO_2$ | $N(CH_3)_4{}^+$ |
| 3-CN | Cl | $CF_3$ | $NO_2$ | $N(C_4H_9)_4{}^+$ |
| 3-CN | Cl | $CF_3$ | $NO_2$ | $C_6H_5\text{-}NH(CH_3)_2{}^+$ |
| 3-CN | Cl | $CF_3$ | $NO_2$ | $NH(C_2H_5)_3{}^+$ |
| 3-CN | H | $NO_2$ | $CF_3$ | $K^+$ |
| 3-CN | H | $NO_2$ | $CF_3$ | $N(CH_3)_4{}^+$ |
| 3-CN | H | $NO_2$ | $CF_3$ | $N(C_4H_9)_4{}^+$ |
| 5-CN | Cl | $CF_3$ | $NO_2$ | $K^+$ |
| 5-CN | Cl | $CF_3$ | $NO_2$ | $N(C_4H_9)_4{}^+$ |
| 5-CN | H | $NO_2$ | $CF_3$ | $N(C_4H_9)_4{}^+$ |
| 3-Cl, 5-CN | Cl | $CF_3$ | $NO_2$ | $K^+$ |
| 3-Cl, 5-CN | Cl | $CF_3$ | $NO_2$ | $N(CH_3)_4{}^+$ |
| 3-Cl, 5-CN | Cl | $CF_3$ | $NO_2$ | $N(C_4H_9)_4{}^+$ |
| 3-Cl, 5-CN | Cl | $CF_3$ | $NO_2$ | $NH_2(i\text{-}C_3H_7)_2$ |
| 3-Cl, 5-CN | Cl | $CF_3$ | $NO_2$ | $(CH_3)_3NCH_2CH_2OH^+$ |
| 3-Cl, 5-CN | H | $NO_2$ | $CF_3$ | $K^+$ |
| 3-Cl, 5-CN | H | $NO_2$ | $CF_3$ | $N(C_4H_9)_4{}^+$ |
| 3-Cl, 5-CN | H | $NO_2$ | $CF_3$ | $NH_2(i\text{-}C_3H_7)_2{}^+$ |
| 3-Br, 5-CN | Cl | $CF_3$ | $NO_2$ | $N(C_4H_9)_4{}^+$ |
| 3-Br, 5-CN | H | $NO_2$ | $CF_3$ | $N(C_4H_9)_4{}^+$ |
| 5-Cl, 3-CN | Cl | $CF_3$ | $NO_2$ | $K^+$ |
| 5-Cl, 3-CN | Cl | $CF_3$ | $NO_2$ | $N(C_4H_9)_4{}^+$ |
| 5-Cl, 3-CN | H | $NO_2$ | $CF_3$ | $K^+$ |
| 5-Cl, 3-CN | H | $NO_2$ | $CF_3$ | $N(C_4H_9)_4{}^+$ |
| 5-Br, 3-CN | Cl | $CF_3$ | $NO_2$ | $N(C_4H_9)_4{}^+$ |
| 5-Br, 3-CN | H | $NO_2$ | $CF_3$ | $N(C_4H_9)_4{}^+$ |
| 6-Cl, 3-CN | Cl | $CF_3$ | $NO_2$ | $N(C_4H_9)_4{}^+$ |
| 6-Cl, 3-CN | H | $NO_2$ | $CF_3$ | $N(C_4H_9)_4{}^+$ |

Tabelle D (Fortsetzung)

| $X_m$/CN | $R^1$ | $R^2$ | $R^3$ | $M^+$ |
|---|---|---|---|---|
| 4-$CH(CH_3)_2$, 6-$OC_2H_5$, 3,5-$(CN)_2$ | Cl | $CF_3$ | $NO_2$ | $N(C_4H_9)_4^+$ |
| 4-$CH(CH_3)_2$, 6-$OC_2H_5$, 3,5-$(CN)_2$ | H | $NO_2$ | $CF_3$ | $N(C_4H_9)_4^+$ |
| 6-$CH_3$, 3,5-$(CN)_2$ | Cl | $CF_3$ | $NO_2$ | $N(C_4H_9)_4^+$ |
| 6-$CH_3$, 3-CN | Cl | $CF_3$ | $NO_2$ | $N(C_4H_9)_4^+$ |
| 6-cyclo-$C_3H_5$, 3-CN | Cl | $CF_3$ | $NO_2$ | $N(C_4H_9)_4^+$ |
| 6-Cl, 4-CN | Cl | $CF_3$ | $NO_2$ | $N(C_4H_9)_4^+$ |
| 6-Cl, 4-CN | H | $NO_2$ | $CF_3$ | $N(C_4H_9)_4^+$ |
| 4,6-$(CH_3)_2$, 3-CN | Cl | $CF_3$ | $NO_2$ | $N(C_4H_9)_4^+$ |
| 4,6-$(CH_3)_2$, 3-CN | H | $NO_2$ | $CF_3$ | $N(C_4H_9)_4^+$ |
| 4,6-$(CH_3)_2$, 3-CN | H | $CH_3$ | $NO_2$ | $N(C_4H_9)_4^+$ |
| 6-$CH(CH_3)_2$, 3-CN | Cl | $CF_3$ | $NO_2$ | $N(C_4H_9)_4^+$ |
| 3-CN,5-Cl,4,6-$(CH_3)_2$ | H | $NO_2$ | $CF_3$ | $K^+$ |
| 3-CN,5-Cl,4,6-$(CH_3)_2$ | H | $NO_2$ | Cl | $K^+$ |
| 3-CN,5-Br,4,6-$(CH_3)_2$ | H | $NO_2$ | $CF_3$ | $K^+$ |
| 3-CN,5-Br,4,6-$(CH_3)_2$ | H | $NO_2$ | Cl | $K^+$ |
| 5-CN,3-Cl,4,6-$(CH_3)_2$ | H | $NO_2$ | $CF_3$ | $K^+$ |
| 5-CN,3-Cl,4,6-$(CH_3)_2$ | H | $NO_2$ | Cl | $K^+$ |
| 5-CN,3-Br,4,6-$(CH_3)_2$ | H | $NO_2$ | $CF_3$ | $K^+$ |
| 5-CN,3-Br,4,6-$(CH_3)_2$ | H | $NO_2$ | Cl | $K^+$ |
| 3-CN,5-Cl,4,6-$(CH_3)_2$ | H | $NO_2$ | $CF_3$ | $N(CH_3)_4^+$ |
| 3-CN,5-Cl,4,6-$(CH_3)_2$ | H | $NO_2$ | Cl | $N(CH_3)_4^+$ |
| 3-CN,5-Br,4,6-$(CH_3)_2$ | H | $NO_2$ | $CF_3$ | $N(CH_3)_4^+$ |
| 3-CN,5-Br,4,6-$(CH_3)_2$ | H | $NO_2$ | Cl | $N(CH_3)_4^+$ |
| 5-CN,3-Cl,4,6-$(CH_3)_2$ | H | $NO_2$ | $CF_3$ | $N(CH_3)_4^+$ |
| 5-CN,3-Cl,4,6-$(CH_3)_2$ | H | $NO_2$ | Cl | $N(CH_3)_4^+$ |
| 5-CN,3-Br,4,6-$(CH_3)_2$ | H | $NO_2$ | $CF_3$ | $N(CH_3)_4^+$ |
| 5-CN,3-Br,4,6-$(CH_3)_2$ | H | $NO_2$ | Cl | $N(CH_3)_4^+$ |
| 3-CN,5-Cl,4,6-$(CH_3)_2$ | H | $NO_2$ | $CF_3$ | $N(n-C_4H_9)_4^+$ |
| 3-CN,5-Cl,4,6-$(CH_3)_2$ | H | $NO_2$ | Cl | $N(n-C_4H_9)_4^+$ |
| 3-CN,5-Br,4,6-$(CH_3)_2$ | H | $NO_2$ | $CF_3$ | $N(n-C_4H_9)_4^+$ |
| 3-CN,5-Br,4,6-$(CH_3)_2$ | H | $NO_2$ | Cl | $N(n-C_4H_9)_4^+$ |
| 5-CN,3-Cl,4,6-$(CH_3)_2$ | H | $NO_2$ | $CF_3$ | $N(n-C_4H_9)_4^+$ |
| 5-CN,3-Cl,4,6-$(CH_3)_2$ | H | $NO_2$ | Cl | $N(n-C_4H_9)_4^+$ |
| 5-CN,3-Br,4,6-$(CH_3)_2$ | H | $NO_2$ | $CF_3$ | $N(n-C_4H_9)_4^+$ |
| 5-CN,3-Br,4,6-$(CH_3)_2$ | H | $NO_2$ | Cl | $N(n-C_4H_9)_4^+$ |

Tabelle D (Fortsetzung)

| $X_m$/CN | $R^1$ | $R^2$ | $R^3$ | $M^+$ |
|---|---|---|---|---|
| 3-CN,5-Cl,4,6-$(CH_3)_2$ | H | $NO_2$ | $CF_3$ | $(i-C_3H_7)_2NHC_2H_5^+$ |
| 3-CN,5-Cl,4,6-$(CH_3)_2$ | H | $NO_2$ | Cl | $(i-C_3H_7)_2NHC_2H_5^+$ |
| 3-CN,5-Br,4,6-$(CH_3)_2$ | H | $NO_2$ | $CF_3$ | $(i-C_3H_7)_2NHC_2H_5^+$ |
| 3-CN,5-Br,4,6-$(CH_3)_2$ | H | $NO_2$ | Cl | $(i-C_3H_7)_2NHC_2H_5^+$ |
| 5-CN,3-Cl,4,6-$(CH_3)_2$ | H | $NO_2$ | $CF_3$ | $(i-C_3H_7)_2NHC_2H_5^+$ |
| 5-CN,3-Cl,4,6-$(CH_3)_2$ | H | $NO_2$ | Cl | $(i-C_3H_7)_2NHC_2H_5^+$ |
| 5-CN,3-Br,4,6-$(CH_3)_2$ | H | $NO_2$ | $CF_3$ | $(i-C_3H_7)_2NHC_2H_5^+$ |
| 5-CN,3-Br,4,6-$(CH_3)_2$ | H | $NO_2$ | Cl | $(i-C_3H_7)_2NHC_2H_5^+$ |
| 3-CN,5-Cl,4,6-$(CH_3)_2$ | H | $NO_2$ | $CF_3$ | $C_6H_6CH_2N(CH_3)_3^+$ |
| 3-CN,5-Cl,4,6-$(CH_3)_2$ | H | $NO_2$ | Cl | $C_6H_6CH_2N(CH_3)_3^+$ |
| 3-CN,5-Br,4,6-$(CH_3)_2$ | H | $NO_2$ | $CF_3$ | $C_6H_6CH_2N(CH_3)_3^+$ |
| 3-CN,5-Br,4,6-$(CH_3)_2$ | H | $NO_2$ | Cl | $C_6H_6CH_2N(CH_3)_3^+$ |
| 5-CN,3-Cl,4,6-$(CH_3)_2$ | H | $NO_2$ | $CF_3$ | $C_6H_6CH_2N(CH_3)_3^+$ |
| 5-CN,3-Cl,4,6-$(CH_3)_2$ | H | $NO_2$ | Cl | $C_6H_6CH_2N(CH_3)_3^+$ |
| 5-CN,3-Br,4,6-$(CH_3)_2$ | H | $NO_2$ | $CF_3$ | $C_6H_6CH_2N(CH_3)_3^+$ |
| 5-CN,3-Br,4,6-$(CH_3)_2$ | H | $NO_2$ | Cl | $C_6H_6CH_2N(CH_3)_3^+$ |

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus

oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomna truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern

(Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 1.001 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 1.027 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 1.037 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 2.009 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 2.021 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).

VI. Man vermischt 90 Gew.-Teile der Verbindung Nr. 3.001 mit 10 Gew.-Teilen N-Methyl-$\alpha$-pyrrolidon und erhält eine Lösung, die zur Anwendung in Form kleinster Tropfen geeignet ist (Wirkstoffgehalt 90 Gew.%).

VII. 20 Gew.-Teile der Verbindung Nr. 4.002 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 4.009 werden mit 3 Gew.-Teilen des Natriumsalzes der Diisobutylnaphthalin-$\alpha$-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 3 kg/ha, vorzugsweise 0,1 bis 2 kg/ha aktive Substanz (a.S.).

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

## Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

### Beispiel 1

Zu einer Suspension von 5,65 g (0,025 Mol) 2-Amino-5-brom-3-cyano-4,6-dimethylpyridin und 7,4 g (0,0275 Mol) 2,4-Dinitro-6-trifluormethyl-chlorbenzol in 60 ml Tetrahydrofuran/tert.-Butanol 1:1 wird bei 0 - 5°C eine Lösung von 5,6 g (0,051 Mol) Kalium-tert.-butylat in 50 ml tert.-Butanol innerhalb 1 h unter heftigem Rühren zugetropft. Nach 1 h bei 0°C wird innerhalb 2 h auf Raumtemperatur (20°C) erwärmt, darauf mit Eisessig angesäuert (pH 4), mit 0,2 l Wasser verdünnt und 3mal mit je 50 ml Diethylether extrahiert. Die vereinigten Etherextrakte werden mit gesättigter NaCl-Lösung gewaschen, über $MgSO_4$ getrocknet, eingeengt und der Rückstand mit Ethanol ausgerührt; man erhält 10,9 g (95 % der Theorie) Verbindung 1.034, gelbe Kristalle, Schmp. 147-149°C

### Beispiel 2

Zu einer Lösung von 3,4 g (0,01 Mol) 3-Chlor-5-cyano-2-(2-nitro-4-trifluormethylanilino)-pyridin (Verbindung 2.016) in 25 ml Tetrahydrofuran werden 0,24 g (0,01 Mol) Natriumhydrid portionsweise zugegeben. Nach 15 Min. werden bei Raumtemperatur 3,5 g (0,02 Mol) Benzolsulfonsäurechlorid zugetropft. Nach 48 h bei 60°C wird mit 150 ml Wasser aufgefüllt und 3mal mit je 100 ml Ether extrahiert. Die vereinigten Etherextrakte werden mit gesättigter NaCl-Lösung gewaschen, über $MgSO_4$ getrocknet und eingeengt. Der Rückstand wird chromatographiert (Kieselgel, Cyclohexan, Essigsäureethylester 9:1). Man erhält 1,5 g (31 % der Theorie), Verbindung 3.001, gelbe Kristalle, Schmp. 147-158°C.

Tabelle 1

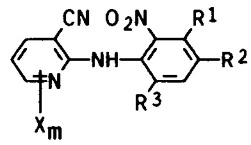

I $R^4$ = H)

| Nr. | Xm | $R^1$ | $R^2$ | $R^3$ | Schmp. |
|---|---|---|---|---|---|
| 1.001 | H | Cl | $CF_3$ | $NO_2$ | 157-158 |
| 1.002 | H | H | $NO_2$ | $CF_3$ | 112-115 |
| 1.003 | 5-Cl | Cl | $CF_3$ | $NO_2$ | 155-156 |
| 1.004 | 5-Cl | H | $CF_3$ | $NO_2$ | 167-168 |
| 1.005 | 5-Cl | Cl | $CO_2C_2H_5$ | $NO_2$ | 216-220 |
| 1.006 | 5-Cl | H | $NO_2$ | $CF_3$ | 153-155 |
| 1.007 | 5-Cl | H | $NO_2$ | Cl | 212-213 |
| 1.008 | 5-Br | Cl | $CF_3$ | $NO_2$ | 136-138 |
| 1.009 | 5-Br | H | $NO_2$ | $CF_3$ | 154-156 |
| 1.010 | 6-Cl | Cl | $CF_3$ | $NO_2$ | 130-134 |
| 1.011 | 6-Cl | H | $NO_2$ | $CF_3$ | 156-159 |
| 1.012 | 6-c-$C_3H_7$ | Cl | $CF_3$ | $NO_2$ | 160 |
| 1.013 | 6-c-$C_3H_7$ | H | $CF_3$ | $NO_2$ | 168-171 |
| 1.014 | 6-c-$C_3H_7$ | H | $NO_2$ | $CF_3$ | 169-173 |
| 1.015 | 6-i-$C_3H_7$ | Cl | $CF_3$ | $NO_2$ | 138-141 |
| 1.016 | 6-i-$C_3H_7$ | H | $CF_3$ | $NO_2$ | 136 |
| 1.017 | 6-i-$C_3H_7$ | H | $NO_2$ | $CF_3$ | 125-131 |
| 1.018 | 6-$CH_3$ | Cl | $CF_3$ | $NO_2$ | 163 |
| 1.019 | 6-$CH_3$ | H | $CF_3$ | $NO_2$ | 204-206 |
| 1.020 | 6-$CH_3$ | H | $NO_2$ | $CF_3$ | 131-132 |
| 1.021 | 4,6-$CH_3$ | Cl | $CF_3$ | $NO_2$ | 165 |
| 1.022 | 4,6-$CH_3$ | H | $CF_3$ | $NO_2$ | 215-216 |
| 1.023 | 4,6-$CH_3$ | H | $NO_2$ | $CF_3$ | 145-152 |
| 1.024 | 4,6-$CH_3$ | H | $C(CH_3)_3$ | $NO_2$ | 192-194 |
| 1.025 | 4,6-$CH_3$ | H | CN | $NO_2$ | 203 (Zers) |
| 1.026 | 4-$CH_3$,6-Cl | Cl | $CF_3$ | $NO_2$ | 148-158 |
| 1.027 | 4-$CH_3$,6-Cl | H | $NO_2$ | $CF_3$ | 128-133 |
| 1.028 | 5-CN,6-$CH_3$ | Cl | $CF_3$ | $NO_2$ | > 220 |
| 1.029 | 5-CN,6-$CH_3$ | H | $NO_2$ | $CF_3$ | 125 |
| 1.030 | 4-$CH_3$,5-CN, 6-$OC_2H_5$ | Cl | $CF_3$ | $NO_2$ | 120 |

Forts. Tab. 1

| Nr. | Xm | $R^1$ | $R^2$ | $R^3$ | Schmp. |
|---|---|---|---|---|---|
| 1.031 | 4-$CH_3$, 5-CN, 6-$OC_2H_5$ | H | $NO_2$ | $CF_3$ | > 220 |
| 1.032 | 4-$CH(CH_3)_3$, 5-CN, 6-$OC_2H_5$ | Cl | $CF_3$ | $NO_2$ | 165-167 |
| 1.033 | 4-$CH(CH_3)_3$, 5-CN, 6-$OC_2H_5$ | Cl | $CO_2C_2H_5$ | $NO_2$ | 173-174 |
| 1.034 | 5-Br, 4, 6- -$(CH_3)_2$ | H | $NO_2$ | $CF_3$ | 147-149 |
| 1.035 | 5-Br, 4, 6- -$(CH_3)_2$ | Cl | $CF_3$ | $NO_2$ | 181-183 |
| 1.036 | 4-$CH(CH_3)_2$, 5-CN, 6-$OC_2H_5$ | H | $NO_2$ | $CF_3$ | 183-185 |
| 1.037 | 4-$CH(CH_3)_2$, 5-CN, 6-$OC_2H_5$ | H | $NO_2$ | Cl | 219-221 |

Tabelle 2

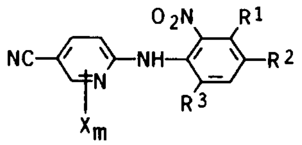

I ($R^4$ = H)

| Nr. | $X_m$ | $R^1$ | $R^2$ | $R^3$ | Schmp (°C) |
|---|---|---|---|---|---|
| 2.001 | H | Cl | $CF_3$ | $NO_2$ | 159-161 |
| 2.002 | H | H | $NO_2$ | $CF_3$ | 141-144 |
| 2.003 | 3-Cl | Cl | $CF_3$ | $NO_2$ | 173-175 |
| 2.004 | 3-Cl | F | $CF_3$ | $NO_2$ | 133-136 |
| 2.005 | 3-Cl | H | $CF_3$ | $NO_2$ | 134-135 |
| 2.006 | 3-Cl | H | CN | $NO_2$ | 210-212 |
| 2.007 | 3-Cl | H | $CO_2CH_3$ | $NO_2$ | 185-187 |
| 2.008 | 3-Cl | Cl | $CO_2C_2H_5$ | $NO_2$ | 182-188 |
| 2.009 | 3-Cl | H | $SO_2CH_3$ | $NO_2$ | >220 |
| 2.010 | 3-Cl | H | $CH_3$ | $NO_2$ | 155 |
| 2.011 | 3-Cl | H | $C(CH_3)_3$ | $NO_2$ | 175-178 |
| 2.012 | 3-Cl | H | $NO_2$ | $CF_3$ | 117-119 |
| 2.013 | 3-Cl | H | $NO_2$ | Cl | 135-138 |
| 2.014 | 3-Cl | H | $NO_2$ | $CH(CH_3)C_2H_5$ | 134-138 |
| 2.015 | 3-Cl | Cl | Cl | Cl | 157-158 |
| 2.016 | 3-Cl | H | $CF_3$ | H | 164-170 |
| 2.017 | 3-Br | H | $NO_2$ | $CF_3$ | 124-126 |
| 2.018 | 4,6-$(CH_3)_2$ | Cl | $CF_3$ | $NO_2$ | >220 |
| 2.019 | 6-Cl | Cl | $CF_3$ | $NO_2$ | 203-204 |
| 2.020 | 6-Cl | H | $NO_2$ | $CF_3$ | 176-185 |
| 2.021 | 3-Br | Cl | $CF_3$ | $NO_2$ | 151-155 |
| 2.022 | 3-Br,4,6-$(CH_3)_2$ | Cl | $CF_3$ | $NO_2$ | 173-175 |
| 2.023 | 3-Br,4,6-$(CH_3)_2$ | H | $NO_2$ | $CF_3$ | 131-135 |

Tabelle 3:

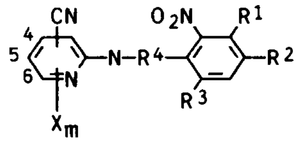

| Nr. | $X_m$ | $R^1$ | $R^2$ | $R^3$ | $R^4$ | Schmp ($^oC$) |
|---|---|---|---|---|---|---|
| 3.001 | 3-Cl,5-CN | H | $CF_3$ | H | $SO_2C_6H_5$ | 147-158 |

Tabelle 4:

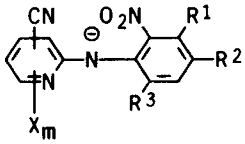

| Nr. | $X_m$/CN | $R^1$ | $R^2$ | $R^3$ | $M^{\oplus}$ | Schmp.($^oC$) |
|---|---|---|---|---|---|---|
| 4.001 | 3-Cl,5-CN | Cl | $CF_3$ | $NO_2$ | $K^{\oplus}$ | 175 |
| 4.002 | 3-Cl,5-CN | Cl | $CF_3$ | $NO_2$ | $N(CH_3)_4^{\oplus}$ | 198 |
| 4.003 | 3-Cl,5-Cn | Cl | $CF_3$ | $NO_2$ | $N(n-C_4H_9)_4^{\oplus}$ | 110-112 |
| 4.004 | 3-Cl,5-CN | Cl | $CF_3$ | $NO_2$ | $NH_2(i-C_3H_7)_2^{\oplus}$ | 92-94 |
| 4.005 | 3-Cl,5-CN | Cl | $CF_3$ | $NO_2$ | $BzlN(CH_3)_3^{\oplus}$ | 135 |
| 4.006 | 3-Cl,5-CN | Cl | $CF_3$ | $NO_2$ | $H_3CN(n-C_4H_9)_3^{\oplus}$ | 60-65 |
| 4.007 | 3-Cl,5-CN | Cl | $CF_3$ | $NO_2$ | $NH(C_2H_5)(i-C_3H_7)_2^{\oplus}$ | 78-82 |
| 4.008 | 3-Cl,5-CN | Cl | $CF_3$ | $NO_2$ | $N(CH_3)_4^{\oplus}$ | 198 |
| 4.009 | 3-Br, 5-CN, 4,6-$(CH_3)_2$ | H | $NO_2$ | $CF_3$ | $K^{\oplus}$ | >230 |
| 4.010 | 3-Br, 5-CN, 4,6-$(CH_3)_2$ | H | $NO_2$ | $CF_3$ | $N(CH_3)_4^{\oplus}$ | 213-220 |
| 4.011 | 3-Br, 5-CN, 4,6-$(CH_3)_2$ | H | $NO_2$ | $CF_3$ | $N(n-C_4H_9)_4^{\oplus}$ | 95-98 |
| 4.012 | 3-CN, 5-Br, 4,6-$(CH_3)_2$ | H | $NO_2$ | $CF_3$ | $N(n-C_4H_9)_4^{\oplus}$ | 94-96 |
| 4.013 | 3-Br, 5-CN | H | $NO_2$ | $CF_3$ | $N(n-C_4H_9)_4^{\oplus}$ | 119-120 |

Anwendungsbeispiele

Die insektizide Wirkung der Verbindungen der allgemeinen Formel I ließ sich durch folgende Versuche zeigen:

Die Wirkstoffe wurden

a) als 0,1 %-ige Lösung in Aceton oder

b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)

aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

A. Aphis fabae (Schwarze Laus), Kontaktwirkung

Stark befallene junge Bohnenpflanzen (Vicia faba) wurden mit der wäßrigen Wirkstoffaufbereitung behandelt.

Nach 24 h wurde die Mortalitätsrate bestimmt.

In diesem Test zeigten die Verbindungen 1.006, 1.009, 1.011, 2.012, 2.017 und 2.023 Wirkschwellen von 20 bis 1000 ppm.

B. Plutella maculipennis (Kohlschaben - Raupe), Kontaktwirkung

Blätter junger Kohlpflanzen wurden mit der wässrigen Wirkstoffaufbereitung benetzt und anschließend auf einen angefeuchteten Filter gelegt. Die präparierten Blätter wurden anschließend mit jeweils 10 Raupen des 4. Entwicklungsstadiums belegt.

Nach 48 h wurde die Mortalitätsrate bestimmt.

In diesem Test zeigten die Verbindungen 1.004, 1.006, 1.009, 1.011, 1.014, 1.017, 1.020, 1.034, 1.036, 1.037, 2.005, 2.012, 2.013 und 2.017 Wirkschwellen von 100 bis 1000 ppm.

C. Tetranychus telarius (Rote Spinne), Kontaktwirkung

Stark befallene getopfte Buschbohnen, die das zweite Folgeblatt paar zeigten, wurden mit wässrigen Wirkstoffaufbereitung tropfnaß gespritzt.

Nach 5 Tagen im Gewächshaus wurde der Bekämpfungserfolg mittels Binokular bestimmt.

In diesem Test zeigten die Verbindungen 1.006, 1.017, 1.020, 1.023, 1.036, 2.012 und 2.013 Wirkschwellen von 20 bis 1000 ppm.

D. Caenorhabditis elegans (Freilebende Nematoden), Kontaktwirkung

Der Boden eines Versuchsgefäßes wurde mit der acetonischen Lösung des Wirkstoffs benetzt und nach dem Abdampfen des Lösungsmittels mit E.-Coli-Bakteriensuspension als Nährmedium bedeckt und mit 50 Nematodensuspension infiziert.

Nach 48 h wurde die Mortalitätsrate bestimmt.

In diesem Test zeigten die Verbindungen 1.002, 1.023, 1.028, 1.029, 1.030, 1.031 und 2.019 Wirkschwellen von 4 bis 100 ppm.

**Patentansprüche**

**1.** Verfahren zu Bekämpfung von Schädlingen, dadurch gekennzeichnet, daß man die Schädlinge und/oder ihren Lebensraum mit einer wirksamen Menge eines 2-Anilino-cyanopyridins der allgemeinen Formel I,

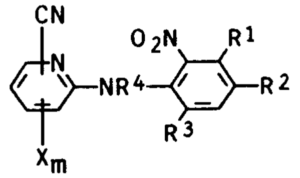

I

behandelt, in der die Substituenten und der Index die folgende Bedeutung haben:

X Nitro; Cyano; Halogen; $C_1$-$C_4$-Alkyl; $C_1$-$C_4$-Halogenalkyl; $C_1$-$C_4$-Alkoxy; $C_3$-$C_6$-Cycloalkyl; $C_1$-$C_4$-Alkylsulfonyl; Phenyl, Phenoxy oder Phenylthio, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

m 1, 2, oder 3, wobei die Reste verschieden sein können, wenn m für 2 oder 3 steht;

$R^1$ Wasserstoff; Halogen; $C_1$-$C_4$-Alkyl; $C_1$-$C_4$-Halogenalkyl; $C_1$-$C_4$-Alkoxy; $C_1$-$C_4$-Halogenalkoxy; Phenoxy oder Phenylthio, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^2$ und $R^3$ unabhängig voneinander

Wasserstoff; Nitro; Cyano; Carboxyl; Halogen; $C_1$-$C_6$-Alkyl; $C_1$-$C_6$-Halogenalkyl; $C_1$-$C_4$-Alkoxy; $C_1$-$C_4$-Halogenalkoxy; $C_1$-$C_4$-Alkylthio; $C_1$-$C_4$-Halogenalkylthio; $C_1$-$C_4$-Alkoxycarbonyl; $C_1$-$C_4$-Alkylaminocarbonyl; Di-$C_1$-$C_4$-alkylaminocarbonyl; $C_1$-$C_4$-Alkylsulfonyl; $C_1$-$C_4$-Alkylaminosulfonyl; Di-$C_1$-$C_4$-alkylaminosulfonyl;

$R^4$ Wasserstoff; Formyl; $C_1$-$C_6$-Alkyl; $C_1$-$C_6$-Halogenalkyl; $C_3$-$C_6$-Alkenyl; $C_3$-$C_6$-Alkinyl; Benzyl, welches seinerseits ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$COR^5$ oder $SO_2R^6$, wobei

$R^5$ für $C_1$-$C_6$-Alkyl; $C_1$-$C_6$-Halogenalkyl; $C_1$-$C_4$-Alkoxy; $C_1$-$C_4$-Halogenalkoxy; $C_1$-$C_4$-Alkylthio; $C_1$-$C_4$-Halogenalkylthio; $C_1$-$C_4$-Alkylamino; Di-$C_1$-$C_4$-alkylamino; Benzyl, Benzyloxy, Benzylthio oder Benzylamino steht, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

und

$R^6$ für $C_1$-$C_6$-Alkyl; $C_1$-$C_6$-Halogenalkyl; Phenyl oder Benzyl steht, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio,

sowie deren landwirtschaftlich brauchbaren Salze.

**2.** Verfahren nach Anspruch 1, dadurch gekennzeichnet, daß man die Schädlinge und/oder ihren Lebensraum mit einer wirksamen Menge eines 2-Anilino-cyanopyridins der allgemeinen Formel I, gemäß Anspruch 1, behandelt, in der die Substituenten und der Index die folgende Bedeutung haben:

$R^1$ Wasserstoff oder Halogen;

$R^2$ und $R^3$ unabhängig voneinander Wasserstoff; Nitro; Cyano; Halogen; $C_1$-$C_4$-Halogenalkyl; $C_1$-$C_4$-Alkoxy oder $C_1$-$C_4$-Halogenalkoxy;

$R^4$ Wasserstoff; $COR^5$ oder $SO_2R^6$, wobei

$R^5$ für $C_1$-$C_6$-Alkyl; $C_1$-$C_6$-Halogenalkyl; $C_1$-$C_4$-Alkoxy; $C_1$-$C_4$-Halogenalkoxy; $C_1$-$C_4$-Alkylthio; $C_1$-$C_4$-Halogenalkylthio; $C_1$-$C_4$-Alkylamino; Di-$C_1$-$C_4$-alkylamino; Benzyl, Benzyloxy, Benzylthio oder Benzylamino steht, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl,

$C_1$-$C_4$-Alkoxy, $C_1$-$C_4$-Halogenalkoxy, $C_1$-$C_4$-Alkylthio und $C_1$-$C_4$-Halogenalkylthio;

und

$R^6$ für Phenyl oder Benzyl steht, wobei die aromatischen Gruppen ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio,

sowie deren landwirtschaftlich brauchbaren Salze.

**3.** Verfahren nach Anspruch 1, dadurch gekennzeichnet, daß man die Schädlinge und/oder ihren Lebensraum mit einer wirksamen Menge eines 2-Anilino-cyanopyridins der allgemeinen Formel I, gemäß Anspruch 1, behandelt, in der die Substituenten und der Index die folgende Bedeutung haben:

$R^1$ Wasserstoff oder Halogen;

$R^2$ und $R^3$ unabhängig voneinander Wasserstoff; Nitro; Cyano; Halogen oder $C_1$-$C_2$-Halogenalkyl;

$R^4$ Wasserstoff oder $SO_2R^6$, wobei

$R^6$ für Phenyl steht, welches ein bis fünf Halogenatome und/oder ein bis drei der

folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio,

sowie deren landwirtschaftlich brauchbaren Salze.

**4.** Verwendung der Verbindungen I gemäß Anspruch 1 zur Bekämpfung von Schädlingen.